(19) Europäisches Patentamt
European Patent Office
Office européen des brevets

(11) Veröffentlichungsnummer: **0 501 280 A2**

# (12) EUROPÄISCHE PATENTANMELDUNG

(21) Anmeldenummer: **92102664.7**

(22) Anmeldetag: **18.02.92**

(51) Int. Cl.[5]: **C07K 5/06**, C07K 5/02, C07D 233/64, A61K 37/64, A61K 31/415

(30) Priorität: **01.03.91 DE 4106488**

(43) Veröffentlichungstag der Anmeldung: **02.09.92 Patentblatt 92/36**

(84) Benannte Vertragsstaaten: **AT BE CH DE DK ES FR GB GR IT LI LU NL SE**

(71) Anmelder: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Juraszyk, Horst, Dr.**
**Kleiner Ring 14**
**W-6104 Seeheim(DE)**
Erfinder: **Raddatz, Peter, Dr.**
**Akazienweg 8A**
**W-6104 Seeheim(DE)**
Erfinder: **Sombroek, Johannes, Dr.**
**Robert-Koch-Strasse 32**
**W-6100 Darmstadt 13(DE)**
Erfinder: **Gante, Joachim, Dr.**
**Stormstrasse 4**
**W-6100 Darmstadt 12(DE)**
Erfinder: **Schmitges Claus J., Dr.**
**Karolingerstrasse 5**
**W-6114 Gross-Umstadt(DE)**
Erfinder: **Minck, Klaus-Otto, Dr.**
**Büchestrasse 8**
**W-6105 Ober-Ramstadt(DE)**

## (54) Renin-inhibierende Peptid-Analoge.

(57) Neue Peptid-Analoga der Formel I

$R^1$-Z-$NR^2$-$CHR^3$-$CR^4$-$CH_2$-$CR^5R^6$-Y   I

worin $R^1$ bis $R^6$, Z und Y die in Patentanspruch 1 angegebenen Bedeutungen haben, sowie ihre Salze hemmen die Aktivität des menschlichen Plasmarenins.

EP 0 501 280 A2

Die Erfindung betrifft neue Peptid-Analoga der Formel I

$R^1$-Z-$NR^2$-$CHR^3$-$CR^4$-$CH_2$-$CR^5R^6$-Y     I

worin

| | |
|---|---|
| $R^1$ | H, $R^7$-O-$C_mH_{2m}$-CO-, $R^7$-$C_mH_{2m}$-O-CO-, $R^7$-$C_mH_{2m}$-CO-, $R^7$-$SO_2$-, $R^8R^9$N-$C_mH_{2m}$-CO-, $R^{10}$-NH-C(=NH)-NH-$C_mH_{2m}$-CO-, $R^8$OOC-$C_mH_{2m}$-CO-, $R^8O_3$S-$C_mH_{2m}$-CO- oder $R^{11}$-$C_mH_{2m}$-$(T)_s$-$(V)_y$-$C_nH_{2n}$-L($R^7$-$C_pH_{2p}$)-$C_rH_{2r}$-CO-, |
| Z | 0 bis 4 peptidartig miteinander verbundene Aminosäurereste ausgewählt aus der Gruppe bestehend aus Abu, Ada, Ala, ßAla, Arg, Asn, Asp, Bia, Cal, Dab, Gln, Glu, Gly, His, N(im)-A-His, Hph, Ile, Isoser, Leu, tert.-Leu, Lys, Mal, Met, αNal, βNal, Mbg, Nle, Nva, Orn, Phe, Pia, Pro, Pya, Ser, Thr, Tia, Tic, Trp, Tyr und Val, |
| Y | -CN, -$NO_2$, -$CH_2$-$NR^{12}R^{13}$, -$CH_2$-$NR^{12}$-$SO_2R^{14}$, -$CH_2$-$NR^{12}$-$COR^{14}$, -$CH_2$-$NR^{12}$-CO-NH-$R^{14}$, -$CH_2$-$NR^{12}$-CS-NH-$R^{14}$ oder -$COR^{16}$, |
| $R^2$, $R^8$, $R^9$ und $R^{13}$ | jeweils H oder A, |
| $R^4$ | (H, $R^{17}$) oder =O, |
| $R^5$ | OH, $OR^7$, $OCOR^7$, $OSiR^{18}R^{19}R^{20}$, $NR^8R^9$ oder -O-(2-tetrahydropyranyl), |
| $R^6$ | H, A, Ar oder Aralkyl, |
| $R^5$ und $R^6$ | zusammen auch (=O), |
| $R^{16}$ | H, OH, $OR^7$, $NR^8R^9$, $OSiR^{18}R^{19}R^{20}$ oder -O-(2-tetrahydropyranyl), |
| $R^{17}$ | OH oder $NH_2$, |
| $R^5$ und $R^{17}$ | zusammen auch -$T^1$-($CR^3R^7$)-$T^2$-, |
| $R^3$, $R^7$, $R^{11}$, $R^{12}$ und $R^{14}$ | jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen, |
| $R^{11}$ | auch $R^8$O-, $R^8R^9$N-, $R^8$OOC- oder $A_3N^{\oplus}$ $An^{\ominus}$, |
| $R^{18}$, $R^{19}$ und $R^{20}$ | jeweils A, Ar oder Aralkyl, |
| $R^{10}$ | H, A oder CN, |
| L | CH oder N, |
| T, $T^1$ und $T^2$ | jeweils O oder $NR^6$, |
| V | $CHOR^2$, CO, S, SO oder $SO_2$, |
| $R^8R^9$N und $NR^{12}R^{13}$ | auch jeweils eine unsubstituierte oder eine durch A, OH, $NH_2$, NHA, $NA_2$, NHAc, NH-CO-$C_xH_{2x}$-O-$R^{15}$, NH-CO-O-$C_xH_{2x}$-$R^{15}$, Hydroxyalkyl, COOH, COOA, $CONH_2$, Aminoalkyl, HAN-alkyl, $A_2$N-alkyl, $A_3N^{\oplus}$ alkyl $An^{\ominus}$, NH-CO-$NH_2$, NH-CO-NHA, Guanidinyl oder Guanidinyl-alkyl substituierte Pyrrolidino-, Piperidino-, Morpholino- oder Piperazinogruppe, |
| $R^{15}$ | A oder Ar-alkyl, |
| s und y | jeweils 0 oder 1, |
| m, n, p, r und x | jeweils 0, 1, 2, 3, 4, 5, 6, 7, 8, 9 oder 10, |
| Ar | unsubstituiertes oder ein- oder mehrfach durch A, OA, Hal, $CF_3$, OH, $NO_2$, Hydroxyalkyl, $NH_2$, NHA, $NA_2$, NHAc, SA, SO-A, $SO_2$-A, $SO_2NH_2$, $SO_2$NHA, COOH, COOA, $CONH_2$, CN, Aminoalkyi, HAN-alkyl, $A_2$N-alkyl, $A_3N^{\oplus}$alkyl $An^{\ominus}$ und/oder Guanidinyl-alkyl substituiertes Phenyl oder unsubstituiertes Naphthyl, |
| Het | einen gesättigten oder ungesättigten 5- oder 6-gliedrigen heterocyciischen Rest mit 1-4 N-, O- und/oder S-Atomen, der mit einem Benzolring kondensiert und/oder ein- oder mehrfach durch A, OA, Hal, $CF_3$, OH, $NO_2$, Carbonylsauerstoff, $NH_2$, NHA, $NA_2$, NHAc, SA, SO-A, $SO_2$-A, $SO_2NH_2$, $SO_2$NHA, COOH, COOA, $CONH_2$, CN, NH-$SO_2$-A, Ar, Ar-alkyl, Ar-alkenyl, Hydroxyalkyl, Aminoalkyl, HAN-alkyl und/oder $A_2$N-alkyl substituiert sein kann und/oder dessen N- und/oder S-Heteroatome auch oxydiert sein könnnen, |
| Hal | F, Cl, Br oder J, |
| Ac | A-CO-, Ar-CO-, Ar-alkyl-CO- oder A-NH-CO-, |
| $An^{\ominus}$ | ein Anion, das auch fehlen kann, wenn stattdessen eine in der Verbindung |

| | |
|---|---|
| | der Formel I enthaltene Carboxygruppe in Form eines Carboxylatanions vorliegt, |
| -alkyl- | eine Alkylengruppe mit 1-8 C-Atomen und |
| A | Alkyl mit 1-8 C-Atomen bedeuten, |

worin ferner an Stelle einer oder mehrerer -NH-CO-Gruppen auch eine oder mehrere -NA-CO-Gruppen stehen können,

mit den Maßgaben, daß

a) im Falle $R^1$ = $R^{11}$-$C_mH_{2m}$-$(T)_s$-V-$C_nH_{2n}$-L($R^7$-$C_pH_{2p}$)-$C_rH_{2r}$-CO- mit V = S, SO oder $SO_2$, und Y = -$CH_2NR^{12}R^{13}$ mit $R^{12}$ = $R^{13}$ = H, $R^5$ dann $OCOR^7$, $OSiR^{18}R^{19}R^{20}$, $NR^8R^9$ oder -O-(2-tetrahydropyranyl) ist,

b) im Falle $R^1$ = $R^{11}$-$C_mH_{2m}$-$(T)_s$-V-$C_nH_{2n}$-L($R^7$-$CH_pH_{2p}$)-$C_rH_{2r}$-CO-mit V = S, SO oder $SO_2$, und Y = $COR^{16}$, $R^{16}$ dann H, OH, $OR^7$, $OSiR^{18}R^{19}R^{20}$ oder -O-(2-tetrahydropyranyl) ist,

sowie deren Salze.

Ähnliche Verbindungen sind aus der EP-OS 03 39 483 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z.B. nach der Methode von F. Fyhrquist et al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z.B. Pepsin und Kathepsin D) sind in der Regel etwa 100 bis 1000mal so hohe Konzentrationen dieser Verbindungen notwendig wie für die Renin-Hemmung. Die Wirkungen der Verbindungen auf den Blutdruck und/oder auf die Herzfrequenz sowie die Hemmung der Reninaktivität im Blutplasma können ferner an wachen Affen, z.B. weiblichen Affen (Macaca fascicularis) ermittelt werden; dabei können Blutdruck und Herzfrequenz in Anlehnung an die Methode von M.J. Wood et al., J. Hypertension 4, 251-254 (1985) gemessen werden. Zur Stimulierung der Reninaktivität werden die Tiere dabei zweckmäßig mit einem Saluretikum vorbehandelt. Blutproben zur Bestimmung der Plasma-Reninaktivität können durch Punktion der Vena femoralis gewonnen werden.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen. Solche diagnostische Tests können ausgeführt werden ähnlich wie es in der EP-A-77 028 angegeben ist.

Die vor- und nachstehend aufgeführten Abkürzungen von Aminosäureresten stehen für die Reste -NR'-R"-CO-, in der Regel -NH-CHR-CO- (worin R, R' und R" die für jede Aminosäure bekannte spezifische Bedeutung haben) folgender Aminosäuren:

| | |
|---|---|
| Abu | 2-Aminobuttersäure |
| Ada | 3-(1-Adamantyl)-alanin |
| Ala | Alanin |
| βAla | β-Alanin |
| Arg | Arginin |
| Asn | Asparagin |
| Asp | Asparaginsäure |
| Bia | 3-(2-Benzimidazolyl)-alanin |
| Cal | 3-Cyclohexylalanin |
| Dab | 2,4-Diaminobuttersäure |
| Gln | Glutamin |
| Glu | Glutaminsäure |
| Gly | Glycin |
| His | Histidin |
| N(im)-A-His | in 1- oder 3-Stellung des Imidazolrings durch A substituiertes Histidin |
| Hph | Homophenylalanin (2-Amino-4-phenyl-buttersäure) |
| Ile | Isoleucin |
| Isoser | Isoserin |
| Leu | Leucin |
| tert.-Leu | tert.-Leucin |
| Lys | Lysin |

| | |
|---|---|
| Mal | 3-(p-Methoxyphenyl)-alanin |
| Met | Methionin |
| αNal | 3-(α-Naphthyl)-alanin |
| βNal | 3-(β-Naphthyl)-alanin |
| Nbg | 2-Norbornyl-glycin |
| Nle | Norleucin |
| Nva | Norvalin |
| N-Me-His | N-Methyl-histidin |
| N-Me-Phe | N-Methyl-phenylalanin |
| Orn | Ornithin |
| Phe | Phenylalanin |
| Pia | 3-(Piperidyl)-alanin [z.B. 2-Pia = 3-(2-Piperidyl)-alanin] |
| Pro | Prolin |
| Pya | 3-(Pyridyl)-alanin [z.B. 3-Pya = 3-(3-Pyridyl)-alanin] |
| Ser | Serin |
| Thr | Threonin |
| Tia | 3-(Thienyl)-alanin [z.B. 2-Tia = 3-(2-Thienyl)-alanin] |
| Tic | 1,2,3,4-Tetrahydroisochinolin-1-carbonsäure |
| Trp | Tryptophan |
| Tyr | Tyrosin |
| Val | Valin. |

Ferner bedeutet nachstehend:

| | |
|---|---|
| BOC | tert.-Butoxycarbonyl |
| BOM | Benzyloxymethyl |
| imi-BOM | Benzyloxymethyl in 1-Stellung des Imidazolrings |
| CBZ | Benzyloxycarbonyl |
| DAPECI | Dimethylaminopropylcarbodiimid |
| DCCI | Dicyclohexylcarbodiimid |
| DMF | Dimethylformamid |
| DNP | 2,4-Dinitrophenyl |
| imi-DNP | 2,4-Dinitrophenyl in 1-Stellung des Imidazolrings |
| ETOC | Ethoxycarbonyl |
| FMOC | 9-Fluorenylmethoxycarbonyl |
| HOBt | 1-Hydroxybenzotriazol |
| IPOC | Isopropoxycarbonyl |
| NMM | N-Methylmorpholin |
| POA | Phenoxyacetyl |
| THF | Tetrahydrofuran. |

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung eines Peptid-Analogen der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

$R^1$-$G^1$-OH II

worin

$G^1$

(a) fehlt,
(b) Z,
(c) $Z^1$ bedeutet

oder eines ihrer reaktionsfähigen Derivate
mit einer Aminoverbindung der Formel III

$H\text{-}G^2\text{-}NR^2\text{-}CHR^3\text{-}CR^4\text{-}CH_2\text{-}CR^5R^6\text{-}Y$ III

worin

$G^2$
(a) Z bedeutet,
(b) fehlt,
(c) $Z^2$ bedeutet und

$Z^1 + Z^2$ zusammen Z bedeuten,

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Aminogruppe durch Behandeln mit einem acylierenden Mittel acyliert und/oder zur Herstellung einer Verbindung der Formel I, $R^4$ = (H, OH) oder (H, $NH_2$), ein Aminoketosäurederivat der Formel I, $R^4$ = O, reduziert oder reduktiv aminiert und/oder einen Rest $R^1$ in einen anderen Rest $R^1$ umwandelt und/oder eine Verbindung der Formel I durch Behandeln mit einer Säure in eines ihrer Salze überführt.

Vor- und nachstehend haben die Reste bzw. Parameter $R^1$ bis $R^{20}$, Z, Y, L, T, $T^1$, $T^2$, V, m, n, p, r, s, x, y, Ar, Het, Hal, Ac, An, A, $G^1$, $G^2$, $Z^1$, und $Z^2$ die bei den Formeln I, II oder III angegebenen Bedeutungen, falls nicht ausdrücklich etwas anderes angegeben ist.

In den vorstehenden Formeln hat A 1-8, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z.B. 1-, 2- oder 3-Methylcyclopentyl, 1-, 2-, 3- oder 4-Methylcyclohexyl.

-Alkyl- bedeutet eine Alkylengruppe mit 1-8 C-Atomen, vorzugsweise mit 1, 2, 3 oder 4 C-Atomen. -Alkyl- bedeutet vorzugsweise Methylen, Ethylen, Propylen, Butylen, ferner auch Pentylen, Hexylen, Heptylen, Octylen, Isopropylen, 1-Ethylpropylen oder 1-, 2- oder 3-Methylbutylen.

Dementsprechend bedeutet Cycloalkylalkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z.B. 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Tricycloalkyl bedeutet vorzugsweise 1-Adamantyl.

Hal bedeutet vorzugsweise F, Cl oder Br, aber auch J.

Ac bedeutet vorzugsweise A-CO-, wie Acetyl, Propionyl oder Butyryl, Ar-CO- wie Benzoyl, o-, m- oder p-Methoxybenzoyl oder 3,4-Dimethoxybenzoyl, A-MH-CO- wie N-Methyl- oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m- oder p-Hydroxhenyl, o-, m- oder p-Sulfamoylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m- oder p-Aminomethylphenyl, o-, m- oder p-Dimethylaminomethylphenyl, o-, m- oder p-Guanidinomethylphenyl, 1- oder 2-Naphthyl.

Dementsprechend bedeutet Ar-alkyl vorzugsweise Benzyl, 1- oder 2-Phenylethyl, o-, m- oder p-Methylbenzyl, 1- oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m- oder -p-Jodphenylethyl, o-, m- oder p-Trifluormethylbenzyl, o-, m- oder p-Hydroxybenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, o-, m- oder p-Aminomethylbenzyl, o-, m- oder p-Dimethylaminomethylbenzyl, o-, m- oder p-Guanidinomethylbenzyl, 1- oder 2-Naphthylmethyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6- 7- oder 8-Chinazolyl. Die heterocyclischen Reste können auch teilweise oder vollständig hydriert sein. Het kann also z.B. auch bedeuten 2,3-Dihydro-2-, -3-, -4- oder -5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-, -3-, -4-, -5-, -6-, -7- oder 8-isochinolyl.

Die heterocyclischen Reste können auch wie angegeben substituiert sein. Het kann z.B. bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 4-Amino-2-methyl-5-pyrimidinyl, 2-Amino-5,6-dimethyl-3-pyrazinyl, 4-Carbamoylpiperidino, ferner z.B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrrolyl, 1-Methyl-4- oder -5-nitro-2-pyrrolyl, 3,5-Dimethyl-4-ethyl-2-pyrrolyl, 4-Methyl-5-pyrazolyl, 5-Methyl-3-isoxazolyl, 3,4-Dimethyl-5-isoxazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 2,6-Dihydroxy-4-pyrimidinyl, 5-Chlor-2-methyl-4-pyrimidinyl, 3-Methyl-2-benzofuryl, 2-Ethyl-3-benzofuryl, 7-Methyl-2-benzothienyl, 1-, 2-, 4-, 5-, 6-oder 7-Methyl-3-indolyl, 1-Methyl-5- oder -6-benzimidazolyl, 1-Ethyl-5- oder -6- benzimidazolyl, 3-, 4-, 5-, 6-, 7- oder 8-Hydroxy-2-chinolyl, 2-Oxo-pyrrolidino, 2-Oxo-piperidino, 2,5-Dioxopyrrolidino, 3-Benzyl-2,5-dioxopyrrolidino.

$R^1$ ist allgemein vorzugsweise $R^8R^9N\text{-}C_mH_{2m}\text{-}CO\text{-}$ oder $R^7\text{-}C_mH_{2m}\text{-}O\text{-}CO\text{-}$, ferner bevorzugt $R^7\text{-}C_mH_{2m}\text{-}CO\text{-}$, $R^8OOC\text{-}C_mH_{2m}\text{-}CO\text{-}$ oder $R^{11}\text{-}C_mH_{2m}\text{-}(T)_s\text{-}(V)_y\text{-}C_nH_{2n}\text{-}L(R^7\text{-}C_pH_{2p})\text{-}C_rH_{2r}\text{-}CO\text{-}$.

Die Gruppe Z besteht vorzugsweise aus einem oder zwei der angegebenen Aminosäurereste; sie kann jedoch auch drei oder vier Aminosäurereste enthalten oder auch fehlen. Vorzugsweise bedeutet Z His, Phe oder Phe-His, ferner bevorzugt Cal-His, Mal-His, αNal-His, βNal-His, Phe-Abu, Phe-Ala, Phe-βAla, Phe-Ser, Phe-Asn, Phe-Gln, Phe-Glu, Phe-Gly, Phe-Nle, Phe-Pya (besonders Phe-3-Pya), Phe-Tia (besonders Phe-3-Tia), Tia-His (besonders 3-Tia-His), Trp-His, Tyr-His oder Pro-Phe-His.

Y ist allgemein vorzugsweise -CN oder $\text{-}CH_2\text{-}NR^{12}\text{-}SO_2R^{14}$, ferner bevorzugt $\text{-}CH_2\text{-}NR^{12}\text{-}CO\text{-}NHR^{14}$ oder $\text{-}CH_2\text{-}NR^{12}\text{-}CS\text{-}NHR^{14}$.

$R^2$, $R^8$, $R^9$ und $R^{13}$ bedeuten jeweils bevorzugt H, ferner bevorzugt Methyl. $R^8R^9N$ ist bevorzugt auch Pyrrolidino, Piperidino, Morpholino, Amino-piperidino wie 4-Aminopiperidino, Alkylaminopiperidino wie 4-Methylaminopiperidino, Dialkylaminopiperidino wie 4-Dimethylaminopiperidino.

$R^3$ ist vorzugsweise Cycloalkylalkyl, insbesondere Cyclohexylmethyl, ferner bevorzugt Alkyl, insbesondere Isobutyl; Ar-alkyl, insbesondere Benzyl; Cycloalkyl, insbesondere Cyclohexyl.

$R^4$ ist vorzugsweise (H, $R^{17}$), worin $R^{17}$ OH oder $NH_2$ bedeutet.

Insbesondere bevorzugt ist $R^4$ (H, OH).

$R^5$ ist vorzugsweise OH, $OR^7$, $OCOR^7$ oder $OSiR^{18}R^{19}R^{20}$.

Vorzugsweise bedeutet $R^5$ zusammen mit $R^{17}$ auch $\text{-}T^1\text{-}(CR^3R^7)\text{-}T^2\text{-}$.

$R^6$ ist vorzugsweise H oder A, insbesondere Methyl.

$R^5$ und $R^6$ bedeuten zusammen auch vorzugsweise (=O).

$R^7$ ist vorzugsweise A, insbesondere Methyl, Ethyl, Isopropyl oder tert.-Butyl; oder Ar, insbesondere Phenyl, 1- oder 2-Naphthyl.

$R^{10}$ ist vorzugsweise H, Methyl oder CN.

$R^{11}$ ist vorzugsweise H; A, insbesondere Methyl oder tert.-Butyl; $R^8R^9N$, insbesondere $A_2N$ wie $(CH_3)_2N$, Piperidino oder 4-Aminopiperidino.

$R^{12}$ und $R^{13}$ sind vorzugsweise H oder A, insbesondere Methyl.

$R^{14}$ ist vorzugsweise A, insbesondere Methyl, Ethyl, Propyl, Isopropyl, Butyl, Isobutyl, sek.-Butyl, tert.-Butyl, Pentyl oder Isopentyl; Cycloalkylalkyl, insbesondere Cyclohexylmethyl; Ar-alkyl, insbesondere Benzyl; Ar, insbesondere Phenyl oder Aminophenyl wie p-Aminophenyl.

$R^{15}$ ist vorzugsweise A mit 1-4 C-Atomen, insbesondere Isopropyl oder tert.-Butyl; oder Ar-alkyl, insbesondere Benzyl.

$R^{16}$ ist vorzugsweise H, OH oder $OR^7$.

$R^{17}$ ist vorzugsweise OH.

$R^{18}$, $R^{19}$ und $R^{20}$ sind jeweils unabhängig voneinander vorzugsweise A, insbesondere Methyl, Ethyl, Propyl, Isopropyl, Butyl, Isobutyl, sek.-Butyl, tert.-Butyl, Pentyl oder Isopentyl; Ar, insbesondere Phenyl oder Ar-alkyl, insbesondere Benzyl.

L ist vorzugsweise CH, ferner bevorzugt N.

T ist vorzugsweise O oder $NR^6$, worin $R^6$ H oder A ist.

$T^1$ und $T^2$ sind vorzugsweise O.

V ist vorzugsweise CO oder $SO_2$.

Der Parameter s ist vorzugsweise 0, aber auch 1; y ist vorzugsweise 1, aber auch 0. Die Summe s + y ist vorzugsweise 1, aber auch 0 oder 2. Der Parameter m ist vorzugsweise 1, 2, 3, 4 oder 5; n ist vorzugsweise 1; p ist vorzugsweise 1; r ist vorzugsweise 1 oder 0. Die Gruppen $C_mH_{2m}$, $C_nH_{2n}$, $C_pH_{2p}$ und $C_rH_{2r}$ sind vorzugsweise geradkettig, bedeuten also vorzugsweise $-(CH_2)_m-$, $-(CH_2)_n-$, $-(CH_2)_p-$ oder $-(CH_2)_r-$.

Dementsprechend bedeutet die Gruppe $R^1$ im einzelnen vorzugsweise $R^8R^9N-(CH_2)_m-CO-$, vor allem $H_2N-C_mH_{2m}-CO-$ wie Aminocarbonyl, Aminoacetyl (H-Gly-), 3-Aminopropionyl (H-βAla-), 4-Aminobutyryl, 5-Aminopentanoyl, 6-Aminohexanoyl, 7-Aminoheptanoyl, 8-Aminooctanoyl, 9-Aminononanoyl, 10-Aminodecanoyl,11-Aminoundecanoyl, aber auch z.B. 2-Amino-propionyl (Ala), 2-Amino-2-methylpropionyl; $ANH-C_mH_{2m}-CO-$ wie Methylaminocarbonyl, Methylaminoacetyl (Sarcosyl), 3-Methylaminopropionyl, 4-Methylaminobutyryl, 5-Methylaminopentanoyl, 6-Methylaminohexanoyl, 6-Ethylaminohexanoyl, 7-Methylaminoheptanoyl, 8-Methylaminooctanoyl, 9-Methylaminononanoyl, 10-Methylaminodecanoyl, 11-Methylaminoundecanoyl; $A_2N-C_mH_{2m}-CO-$wie Dimethylaminocarbonyl, Dimethylaminoacetyl, 3-Dimethylaminopropionyl, 4-Dimethylaminobutyryl, 5-Dimethylaminopentanoyl, 6-Dimethylaminohexanoyl, 6-Diethylaminohexanoyl, 7-Dimethylaminoheptanoyl, 8-Dimethylaminooctanoyl, 9-Dimethylaminononanoyl, 10-Dimethylaminodecanoyl, 11-Dimethylaminoundecanoyl; $A-O-CO-NH-C_mH_{2m}-CO-$ wie BOC-Gly, ETOC-Gly-, IPOC-Gly, BOC-βAla, ETOC-βAla, IPOC-βAla-, 4-BOC-amino-butyryl, 5-BOC-amino-pentanoyl, 6-BOC-amino-hexanoyl, 7-BOC-amino-heptanoyl, 8-BOC-amino-octanoyl, 9-BOC-amino-nonanoyl, 10-BOC-amino-decanoyl, 11-BOC-amino-undecanoyl; $ArCH_2-O-CO-NH-C_mH_{2m}-CO-$ wie CBZ-Gly-, CBZ-βAla, 4-CBZ-amino-butyryl, 5-CBZ-amino-hexanoyl, 7-CBZ-amino-heptanoyl, 8-CBZ-amino-octanoyl, 9-CBZ-aminononanoyl, 10-CBZ-amino-decanoyl, 11-CBZ-amino-undecanoyl; Pyrrolidino-$C_mH_{2m}$-CO- wie Pyrrolidinocarbonyl, Pyrrolidino-acetyl, 3-Pyrrolidino-propionyl, 4-Pyrrolidinobutyryl, 5-Pyrrolidino-pentanoyl, 6-Pyrrolidino-hexanoyl, 4-Pyrrolidino-heptanoyl, 8-Pyrrolidino-octanoyl, 9-Pyrrolidino-nonanoyl, 10-Pyrrolidino-decanoyl; Piperidino-$C_mH_{2m}$-CO- wie Piperidinocarbonyl, Piperidinoacetyl, 3-Piperidino-propionyl, 4-Piperidino-butyryl, 5-Piperidino-pentanoyl, 6-Piperidinohexanoyl, 7-Piperidinoheptanoyl, 8-Piperidino-octanoyl, 9-Piperidino-nonanoyl, 10-Piperidino-decanoyl; Morpholino-$C_mH_{2m}$-CO wie Morpholinocarbonyl, Morpholinoacetyl, 3-Morpholino-propionyl, 4-Morpholinobutyryl, 5-Morpholino-pentanoyl, 6-Morpholino-hexanoyl, 7-Morpholino-heptanoyl, 8-Morpholino-octanoyl, 9-Morpholino-nonanoyl, 10-Morpholino-decanoyl; 4-Amino-piperidino-$C_mH_{2m}$-CO- wie 4-Amino-piperidino-carbonyl, 4-Amino-piperidino-acetyl, 3-(4-Amino-piperidino)-propionyl, 4-(4-Amino-piperidino)-butyryl, 5-(4-Amino-piperidino)-pentanoyl, 6-(4-Amino-piperidino)-hexanoyl, 7-(4-Amino-piperidino)-heptanoyl, 8-(4-Amino-piperidino)-octanoyl, 9-(4-Amino-piperidino)-nonanoyl, 10-(4-Amino-piperidino)-decanoyl; 4-Dialkylamino-piperidino-$C_mH_{2m}$-CO- wie 4-Dimethylamino-piperidinocarbonyl, 4-Dimethylamino-piperidino-acetyl; 4-Guanidino-piperidino-$C_mH_{2m}$-CO- wie 4-Guanidino-piperidino-carbonyl, 4-Guanidino-piperidino-acetyl; 4-Carboxy-piperidino-$C_mH_{2m}$-CO- wie 4-Carboxy-piperidino-carbonyl, 4-Carboxy-piperidino-acetyl; 4-Alkoxycarbonyl-piperidino-$C_mH_{2m}$-CO- wie 4-Methoxycarbonyl-piperidino-carbonyl, 4-Ethoxycarbonyl-piperidino-carbonyl, 4-Methoxycarbonyl-piperidino-acetyl, 4-Ethoxycarbonyl-piperidino-acetyl; 4-AcNH-piperidino-$C_mH_{2m}$-CO- wie 4-Acetamido-piperidino-carbonyl, 4-Acetamido-piperidino-acetyl; $H_2N-C(=NH)-NH-C_mH_{2m}-CO-$ wie Guanidinoacetyl, 3-Guanidino-propionyl, 4-Guanidino-butyryl, 5-Guanidino-pentanoyl, 6-Guanidino-hexanoyl, 7-Guanidino-heptanoyl, 8-Guanidino-octanoyl; $NC-NH-C(=NH)-NH-C_mH_{2m}-CO-$ wie N'-

Cyanguanidino-acetyl, 3-(N'-Cyanguanidino)-propionyl, 4-(N'-Cyanguanidino)-butyryl, 5-(N'-Cyanguanidino)-pentanoyl, 6-(N'-Cyanguanidino)-hexanoyl, 7-(N'-Cyanguanidino)-heptanoyl, 8-(N'-Cyanguanidino)-octanoyl; $HOOC-C_mH_{2m}-CO-$ wie Malonyl, Succinyl, Glutaryl, Adipyl, 6-Carboxyhexanoyl, 7-Carboxyheptanoyl, 8-Carboxyoctanoyl, 9-Carboxynonanoyl, 10-Carboxy-decanoyl, 11-Carboxyundecanoyl; $AOOC-C_mH_{2m}-CO-$ wie Methoxycarbonyl-acetyl, 3-Methoxycarbonyl-propionyl, 4-Methoxycarbonyl-butyryl, 5-Methoxycarbonyl-pentanoyl, 6-Methoxycarbonyl-hexanoyl, 7-Methoxycarbonyl-heptanoyl, 8-Methoxycarbonyl-octanoyl, 9-Methoxycarbonyl-nonanoyl, 10-Methoxycarbonyl-decanoyl, Ethoxycarbonyl-acetyl, 3-Ethoxycarbonyl-propionyl, 4-Ethoxycarbonyl-butyryl, 5-Ethoxycarbonyl-pentanoyl, 6-Ethoxycarbonyl-hexanoyl, 7-Ethoxycarbonyl-heptanoyl, 8-Ethoxycarbonyl-octanoyl, 9-Ethoxycarbonyl-nonanoyl, 10-Ethoxycarbonyl-decanoyl; $H-SO_3-C_mH_{2m}-CO-$ wie Sulfo-acetyl, 3-Sulfo-propionyl, 4-Sulfo-butyryl, 5-Sulfo-pentanoyl, 6-Sulfo-hexanoyl, 7-Sulfo-heptanoyl, 8-Sulfo-octanoyl, 9-Sulfo-nonanoyl, 10-Sulfo-decanoyl; $A-SO_3-C_mH_{2m}-CO$ wie Methoxysulfonyl-acetyl, 3-Methoxysulfonyl-propionyl, 4-Methoxysulfonyl-butyryl, 5-Methoxysulfonyl-pentanoyl, 6-Methoxysulfonyl-hexanoyl, 7-Methoxysulfonyl-heptanoyl, 8-Methoxysulfonyl-octanoyl, 9-Methoxysulfonyl-nonanoyl, 10-Methoxysulfonyl-decanoyl, Ethoxysulfonyl-acetyl, 3-Ethoxysulfonyl-propionyl, 4-Ethoxysulfonyl-butyryl, 5-Ethoxysulfonyl-pentanoyl, 6-Ethoxysulfonyl-hexanoyl, 7-Ethoxysulfonyl-heptanoyl, 8-Ethoxysulfonyl-octanoyl, 9-Ethoxysulfonyl-nonanoyl, 10-Ethoxysulfonyl-decanoyl; $R^{11}-C_mH_{2m}-CO-C_nH_{2n}-CH(R^7-C_pH_{2p})-C_rH_{2r}-CO-$, vor allem $A-CO-CH_2-CH(Ar-CH_2)-CO-$ wie 2-Benzyl-4-oxo-5,5-dimethylhexanoyl, 2-(1-Naphthylmethyl)-4-oxo-5,5-dimethyl-hexanoyl; ferner $R^8R^9N-CO-CH_2-CH(Ar-CH_2)-CO-$wie 2-Benzyl-3-(4-aminopiperidinocarbonyl)-propionyl, 2-(1-Naphthylmethyl)-3-(4-aminopiperidinocarbonyl)-propionyl; $R^{11}-C_mH_{2m}-SO_2-C_nH_{2n}-CH(R^7-C_pH_{2p})-C_rH_{2r}-CO-$, vor allem $A-SO_2-CH_2-CH(Ar-CH_2)-CO$ wie 2-Benzyl-3-tert.-butylsulfonylpropionyl,2-(1-Naphthylmethyl)-3-tert.-butylsulfonylpropionyl; $R^{11}-C_mH_{2m}-NH-CO-C_nH_{2n}-CH-(R^7-C_pH_{2p})-C_rH_{2r}-CO-$, vor allem $R^8R^9N-(CH_2)_m-NH-CO-CH_2-CH(Ar-CH_2)-CO-$ wie 2-Benzyl-3-(N-3-dimethylaminopropyl-carbamoyl)-propionyl, 2-(1-Naphthylmethyl)-3-(N-3-dimethylaminopropyl-carbamoyl)-propionyl,2-Benzyl-3-(N-5-dimethylaminopentyl-carbamoyl)-propionyl; $A-CH(R^7-C_pH_{2p})-C_rH_{2r}-CO-$, vor allem $A-CH(Ar-CH_2)-CO-$ wie 2-Benzylhexanoyl, 2-Benzyl-heptanoyl; $R^{11}-C_mH_{2m}-NH-CO-$, vor allem $R^8R^9N-(CH_2)_m-NH-CO$ wie N-3-Dimethylaminopropyl-carbamoyl, N-5-Dimethylaminopentyl-carbamoyl; $R^{11}-C_mH_{2m}-N(R^7-C_pH_{2p})-C_rH_{2r}-CO-$, vor allem $A-N(Ar-CH_2)-CO-$ wie N-Benzyl-N-butyl-carbamoyl, N-Benzyl-N-isopentyl-carbamoyl; $R^7-C_mH_{2m}-O-CO-$, vor allem A-O-CO- wie ETOC, IPOC, BOC sowie $Ar-C_mH_{2m}-O-CO$ wie CBZ; $R^7-C_mH_{2m}-CO-$ wie 3,3-Dimethylbutyryl.

Die Gruppe Y bedeutet dementsprechend im einzelnen vorzugsweise -CN; $-CH_2NH-SO_2R^{14}$, vor allem $-CH_2-NH-SO_2-A$ wie Methan-, Ethan-, Propan-, 2-Methylpropan-, Butan-sulfonamidomethyl, ferner z.B. Phenylmethan-, Cyclohexylmethan-sulfonamidomethyl; $-CH_2-NH-CO-NH-R^{14}-$, vor allem $-CH_2-NH-CO-NH-A$ wie N'-Methyl-ureido-methyl, N'-Propyl-ureido-methyl, N'-Butyl-ureido-methyl, N'-Isopentyl-ureido-methyl, ferner $-CH_2-NH-CO-NH-Ar$ wie N'-Phenyl-ureido-methyl, N'-p-Aminophenyl-ureido-methyl; $-CH_2-NH-CS-MH-R^{14}$, vor allem $-CH_2-NH-CS-NH-A$ wie N'-Methyl-thioureido-methyl;$-CH_2-NH-COR^{14}$, vor allem $-CH_2-NH-CO-A$wie Acetamidomethyl, Propionamidomethyl, Butyramidomethyl.

Falls $R^1 = R^{11}-C_mH_{2m}-(T)_s-V-C_nH_{2n}-L(R^7-C_pH_{2p})-C_rH_{2r}-CO-$mit V = S, SO oder $SO_2$ und gleichzeitig Y = $-CH_2-NH_2$,so bedeutet $R^5$ $OCOR^7$, $OSiR^{18}R^{19}R^{20}$, $NR^8R^9$ oder -O-(2-tetrahydropyranyl).

Ferner bedeutet $R^{16}$ H, OH, $OR^7$, $OSiR^{10}R^{19}R^{20}$ oder -O-(2-tetrahydropyranyl), falls $R^1 = R^{11}-C_mH_{2m}-(T)_s-V-C_nH_{2n}-L(R^7-C_pH_{2p})-C_rH_{2r}-CO-$ mit V = S, SO oder $SO_2$ und Y $COR^{16}$ bedeutet.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen, wobei die S-Enantiomeren im allgemeinen bevorzugt sind.

Die oben erwähnten Cycloalkyl- und Phenylgruppen sind vorzugsweise unsubstituiert oder tragen vorzugsweise 1 bis 3, insbesondere 1 oder 2 Substituenten.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ik ausgedrückt werden:

Ia $R^7-C_mH_{2m}-O-CO-Z-NH-CHR^3-CHOH-CH_2-CHOH-Y$;

Ib $R^7-C_mH_{2m}-CO-Z-NH-CHR^3-CHOH-CH_2-CHOH-Y$;

Ic $R^8R^9N-C_mH_{2m}-CO-Z-NH-CHR^3-CHOH-CH_2-CHOH-Y$;

Id $R^{10}-NH-C(=NH)-NH-C_mH_{2m}-CO-Z-NH-CHR^3-CHOH-CH_2-CHOH-Y$;

Ie $R^8OOC-C_mH_{2m}-CO-Z-NH-CHR^3-CHOH-CH_2-CHOH-Y$;

If 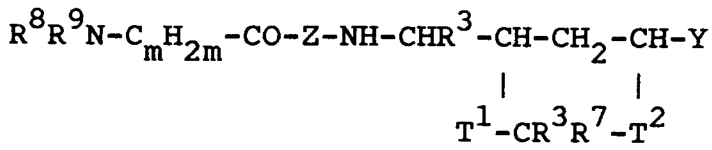

Ig $R^{11}-C_mH_{2m}-(T)_s-(V)_y-C_nH_{2n}-L(R^7-C_pH_{2p})-C_rH_{2r}-CO-Z-NH-CHR^3-CHOH-CH_2-CHR^5-Y$;

Ih 4-Aminopiperidinocarbonyl-Z-NH-$CHR^3-CHOH-CH_2-CHOH-Y$;

Ii $A-CO-CH_2-CH(ArCH_2)-CO-Z-NH-CHR^3-CHOH-CH_2-CHOH-Y$;

Ij $A-SO_2-CH_2-CH(ArCH_2)-CO-Z-NH-CHR^3-CHOH-CH_2-CHR^5-Y$;

Ik $R^8R^9N-C_mH_{2m}-NH-CO-CH_2-CH(ArCH_2)-CO-Z-NH-CHR^3-CHOH-CH_2-CHR^5-Y$.

Insbesondere bevorzugt sind Verbindungen der Teilformeln:

(a) Iaa bis Ika, die den Formeln Ia bis Ik entsprechen, worin jedoch zusätzlich

Z His, Phe, Phe-$\beta$Ala, Phe-Ser oder Phe-His bedeutet;

(b) Iab bis Ikb sowie Iaab bis Ikab, die den Formeln Ia bis Ik sowie Iaa bis Ika entsprechen, worin jedoch zusätzlich

$R^3$ Isobutyl oder Cyclohexylmethyl bedeutet.

Insbesondere sind bevorzugt Verbindungen der Teilformeln:

I* sowie Ia* bis Ik*, die den Formeln I sowie Ia bis Ik sowie solche Verbindungen, die den anderen vorstehend genannten Teilformeln entsprechen, worin jedoch zusätzlich

Y -CN, $-CH_2-NH-CO-NH-R^{14}$, $-CH_2-NH-CO-R^{14}$ oder $-CH_2-NH-SO_2-R^{14}$ und

$R^{14}$ A, Ar-alkyl oder Cycloalkyl-alkyl bedeuten;

I' sowie Ia' bis Ik', die den Formeln I sowie Ia bis Ik sowie solche Verbindungen, die den anderen vorstehend genannten Teilformeln entsprechen, worin jedoch zusätzlich

Y $-CH_2-NH-SO_2-A$ bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A-45665, EP-A-77028, EP-A-77029, EP-A-81783, EP-A-249096) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z.B. solche die der Formel I entsprechen, aber an Stelle einer His-Gruppe eine N(im)-R'-His-Gruppe (worin R' eine Aminoschutzgruppe bedeutet, z.B. BOM oder DNP) enthalten, oder solche der Formel $R^1-Z-NR^2-CHR^3-CH(NHR')-CH_2-CR^6-(NHR')-Y$.

Ferner sind Ausgangsstoffe bevorzugt, die an Stelle des H-Atoms einer Hydroxygruppe eine Hydroxyschutzgruppe tragen, z.B. solche der Formel $R^1-Z-NR^2-CHR^3-CHOR''-CH_2-CR^6OR''-Y$, worin R'' eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht

entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. DMP), Aralkoxymethyl- (z.B. BOM) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, ETOC, 2,2,2-Trichlorethoxycarbonyl, IPOC, BOC, 2-Jodethoxycarbonyl; Aralkyloxycarbonyl wie CBZ, 4-Methoxybenzyloxycarbonyl, FMOC. Bevorzugte Aminoschutzgruppen sind BOC, DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird Vorzugsweise im Überschuß ohne Zusatz eines weiteren Lösungsmittels vervendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°, Vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-30° abgespalten weden, die FMOC-Gruppe mit einer etwa 5-20%igen Lösung Von Dimethylamin, Diethylamin oder Piperidin in DMF bei 15-30°. Eine Abspaltung der DNP-Gruppe gelingt z.B. auch mit einer etwa 3-10%igen Lösung Von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können auch durch direkte Peptidsynthese aus einer Carbonsäure- (Formel II) und einer Aminkomponente (Formel III) erhalten werden. Als Carbonsäurekomponenten eignen sich z.B. solche der Teilformeln (a) $R^1$-OH, (b) $R^1$-Z-OH, als Aminkomponenten solche der Teilformeln (a) H-Z-$NR^2$-$CHR^3$-$CR^4$-$CH_2$-$CR^5R^6$-Y, (b) H-$NR^2$-$CHR^3$-$CR^4$-$CH_2$-$CR^5R^6$-Y. Die Peptidbindung kann aber auch innerhalb der Gruppe Z geknüpft werden; dabei wird eine Carbonsäure der Formel $R^1$-$Z^1$-OH mit einer Aminoverbindung der Formel H-$Z^2$-$NR^2$-$CHR^3$-$CR^4$-$CH_2$-$CR^5R^6$-Y umgesetzt, wobei $Z^1 + Z^2 = Z$ ist. Dabei arbeitet man zweckmäßig nach üblichen Methoden der Peptid-Sypthese, wie sie z.B. in Houben-Weyl, 1.c., Band 15/II, Seiten 1-806 (1974) beschrieben sind.

Die Reaktion gelingt vorzugsweise in Gegenwart eines Dehydratisierungsmittels, z.B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle Von II bzw. III können auch geeignete reaktionsfähige Derivate dieser Stoffe in die Reaktion eingesetzt werden, z.B. solche, in denen reaktive Gruppen intermediär durch Schutzgruppen blockiert sind. Die Säurederivate II können z.B. in Form ihrer aktivierten Ester verwendet werden, die zweckmäßig in situ gebildet werden, z.B. durch Zusatz von HOBt oder N-Hydroxysuccinimid.

Harnstoffderivate der Formel I [$R^1$ = $R^8$-NH-CO- oder $R^{11}$-$C_mH_{2m}$-$(T)_s$-$(V)_y$-$C_nH_{2n}$-NH-CO] sind z.B erhältlich, indem man ein entsprechendes Isocyanat (z.B. der Formel $R^8$-NCO; herstellbar aus einem Amin der Formel $R^8$-$NH_2$ und Phosgen) mit einem Amin der Formel H-Z-$NR^2$-$CHR^3$-$CR^4$-$CH_2$-$CR^5R^6$-Y (IIa) umsetzt, zweckmäßig in einem inerten Lösungsmittel wie THF bei Temperaturen zwischen etwa -10 und 40°, vorzugsweise zwischen 10 und 30°.

Die Ausgangsstoffe der Formeln II und III sind großenteils bekannt. Sofern sie nicht bekannt sind, können sie nach bekannten Methoden, z.B. den oben angegebenen Methoden der Peptidsynthese und der Abspaltung von Schutzgruppen hergestellt werden.

Die Aminkomponente der Formel IIIb kann beispielsweise nach folgendem allgemeinen Schema erhalten werden.

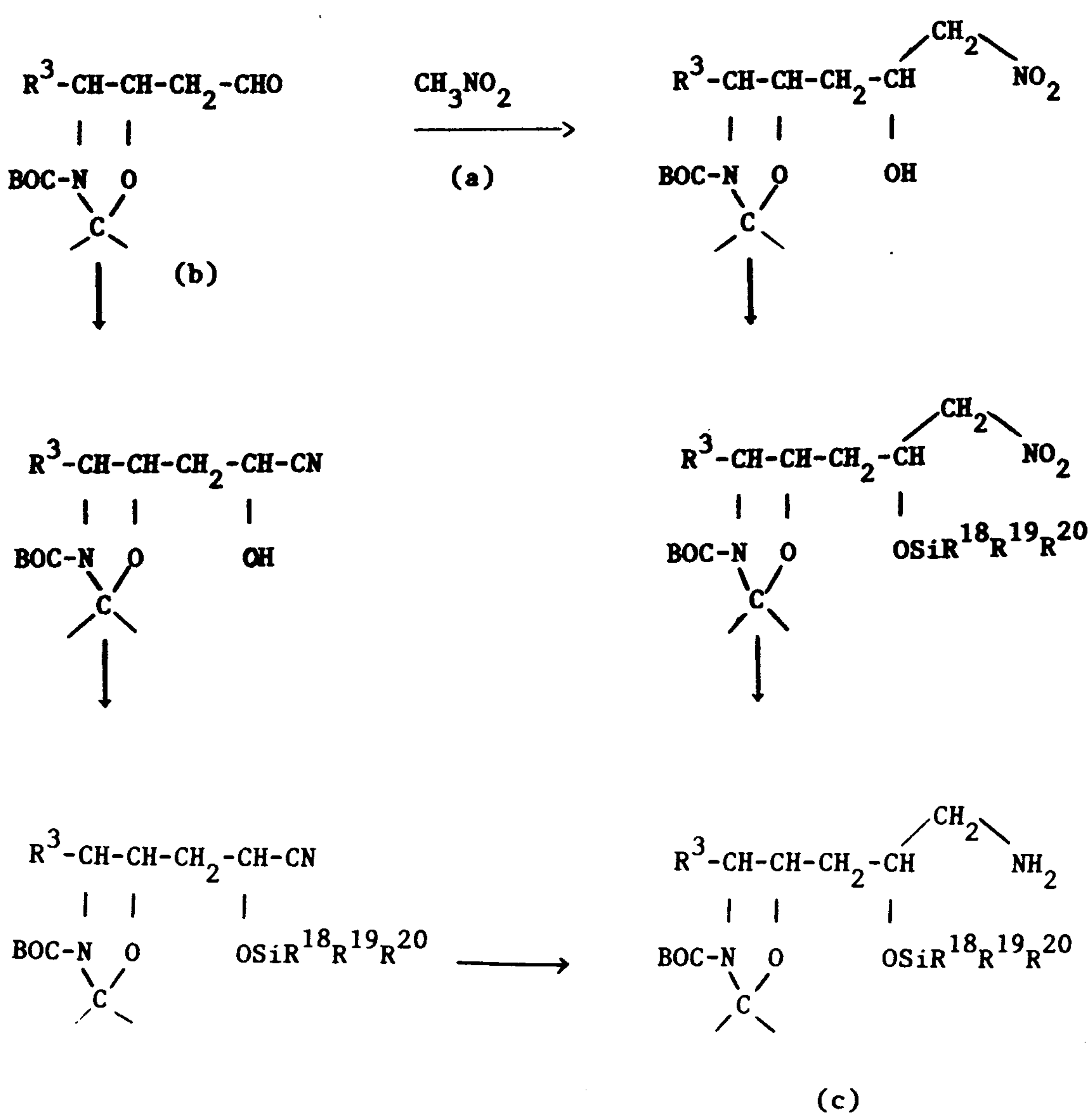

Die Verbindung (c) kann dann nach Belieben nach bekannten Methoden am Substituent $CH_2NH_2$ - (entspricht Y) variiert werden. Nach Abspalten der Silylgruppe, der BOC-Schutzgruppe und Spaltung des Oxazolidinylringes erhält man beispielsweise die Aminkomponente $H_2N$-$CHR^3$-CHOH-$CH_2$-CHOH-$CH_2NH_2$. Die Peptidkopplung mit z.B. $R^1$-Z-OH erfolgt dann anschließend nach üblichen Methoden.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann z. B. eine Verbindung der Formel I, die eine $R^{15}$-$C_xH_{2x}$-O-CO-NH-, eine AcNH-, eine $ArCH_2$-$SO_3$- oder eine AOOC-Gruppe enthält, in die entsprechende Verbindung der Formel I umgewandelt werden, die stattdessen eine $H_2N$-, eine $HSO_3$- oder eine HOOC-Gruppe enthält, zweckmäßig durch selektive Solvolyse nach einer der oben angegebenen Methoden. AOOC-Gruppen können z.B. mit NaOH oder KOH in Wasser-Dioxan bei Temperaturen zwischen 0 und 40°, Vorzugsweise 10 und 30°, verseift werden.

Es ist auch möglich, eine Verbindung der Formel I, die eine freie primäre oder sekundäre Aminogruppe enthält, zu acylieren. So kann man insbesondere Verbindungen der Formel I, in denen Y eine $CH_2$-NH-$R^{12}$-Gruppe bedeutet, mit acylierenden Mitteln der Formeln $R^{14}$-$SO_2Cl$, $R^{14}$-COCl oder $R^{14}$-NCO umsetzen, zweckmäßig in Gegenwart eines inerten Lösungsmittels wie THF und/oder einer Base wie Pyridin oder Triethylamin bei Temperaturen zwischen -10 und +30°.

Weiterhin können Ketoverbindungen der Formel I ($R^4$ = O und/oder $R^5R^6$ = O) zu Verbindungen der Formel I ($R^4$ = (H, OH) und/oder $R^5R^6$ = H, OH) reduziert werden, beispielsweise mit einem komplexen Metallhydrid wie $NaBH_4$, das nicht gleichzeitig die Peptid-Carbonylgruppen reduziert, in einem inerten Lösungsmittel wie Methanol bei Temperaturen zwischen etwa -10 und +30°.

Ketoverbindungen der Formel I ($R^4$ = O und/oder $R^5R^6$ = O) können auch durch reduktive Aminierung in Verbindungen der Formel I ($R^4$ = H, $NH_2$ und/oder $R^5R^6$ = H, $NH_2$) übergeführt werden. Man kann ein- oder mehrstufig reduktiv aminieren. So kann man z.B. die Ketoverbindung mit Ammoniumsalzen, z.B. Ammoniumacetat, und $NaCNBH_3$ behandeln, vorzugsweise in einem inerten Lösungsmittel, z.B. einem Alkohol wie Methanol, bei Temperaturen zwischen etwa 0 und 50°, insbesondere zwischen 15 und 30°. Weiterhin ist es möglich, die Ketoverbindung zunächst mit Hydroxylamin in üblicher Weise in das Oxim zu überführen und dieses, z.B. durch katalytische Hydrierung an Raney-Nickel, zum Amin zu reduzieren.

Eine Base der Formel I kann mit einer Säure in das zugehörige Säureadditionssalz übergeführt wrden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich fur die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren Verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-249096 beschriebenen Verbindungen verabreicht, vorzugsweise in Dosierungen zwischen etwa 10 mg und 1 g, insbesondere zwischen 50 und 500 mg pro Dosierungseinheit. Die tägliche Dosierung liegt vorzugsweise zwischen etwa 0,2 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Renin-abhängige Hypertension und Hyperaldosteronismus können wirksam behandelt werden durch Verabfolgung von Dosierungen zwischen insbesondere etwa 1 und 300, vorzugsweise zwischen 5 und 50 mg/kg Körpergewicht. Für diagnostische Zwecke können die neuen Verbindungen zweckmäßig in Einzeldosen zwischen etwa 0,1 und 10 mg/kg Körpergewicht verabreicht werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation.

## Beispiel 1

a) Ein Gemisch von 380 mg (0,45 mMol) N-[5S-(BOC-Phe-(imi-BOM-His)-amino)-6-cyclohexyl-2S,4S-dihydroxyhexyl]-propansulfonsäureamid (F. 159-161°) [erhältlich durch Reaktion von (4S,5S)-3-BOC-4-cyclohexylmethyl-2,2-dimethyl-oxazolidinyl-(5)-acetaldehyd mit NaCN in Gegenwart von Eisessig in THF bei 4° zu 2-[(4S,5S)-3-BOC-4-cyclohexylmethyl-2,2-dimethyloxazolidinyl-(5)]-(1R,S)-1-hydroxy-propionitril; Umsetzung mit tert. Butyldimethylchlorsilan in Gegenwart von Imidazol bei Raumtemperatur zu 2-[-(4S,5S)-3-BOC-4-cyclohexylmethyl-2,2-dimethyloxazolidinyl-(5)]-(1R,S)-1-(trimethylsilyloxy)propionitril; Hydrierung in Methanol an Raney-Ni bei Raumtemperatur zu 3-[(4S,5S)-3-BOC-4-cyclohexylmethyl-2,2-dimethyloxazolidinyl-(5)]-(2R,S)-2-(trimethylsilyloxy)-propylamin; Reaktion mit 1-Propansulfonylchlorid in THF in Gegenwart von Triethylamin bei 10° zu N-[3-{(4S,5S)-3-BOC-4-cyclohexylmethyl-2,2-dimethyloxazolidinyl-(5)}-(2R,S)-2-trimethylsilyloxy-propyl]propansulfonsäureamid, chromatographische Trennung der Diastereomeren; Abspaltung der Trimethylsilylgruppe aus dem 2S-Epimeren durch Umsetzung mit Tetrabutylammoniumfluorid in Ether, Spaltung mit HCl-gesättigtem Essigsäureethylester zu N-(5S-Amino-6-cyclohexyl-2S,4S-dihydroxy-1-hexyl)-propansulfonsäureamid (F. 102-104°); Kondensation mit BOC-(imi-BOM-His)-OH in Gegenwart von HOBt, NMM und DAPECI in DMF zu N-[5S-BOC-(imi-BOM-His)amino-6-cyclohexyl-2S,4S-dihydroxy-1-hexyl)-propansulfonsäureamid (F. 131-133°); Abspaltung der BOC-Gruppe mit HCl/Essigsäureethylester und Kondensation mit BOC-Phe-OH in Gegenwart von HOBt, NMM und DAPECI-Hydrochlorid in DMF], 590 mg (9,3 mMol) Ammoniumformiat und 480 mg Pd-Kohle in 38 ml Methanol wird 20 Stunden bei Raumtemperatur gerübrt. Man filtriert ab, engt die Lösung ein, gibt 27 ml einer 5%igen $NaHCO_3$-Lösung zu, rührt und saugt ab. Nach Aufreinigung der Kristalle erhält man N-[5S-(BOC-Phe-His-amino)-6-cyclohexyl-2S,4S-dihydroxyhexyl]-propansulfonsäureamid, F. 125-127°.

b) Diese und die folgenden Verbindungen kann man ebenso aus den entsprechenden imi-DNP-His-Derivaten durch Abspaltung der DNP-Gruppe auf folgende Weise erhalten: Ein Gemisch des entsprechenden DMP-Derivates mit 2-Mercaptoethanol in DMF und Wasser wird unter Rühren bei 20° mit wäßriger $Na_2CO_3$-Lösung auf pH 8 eingestellt und 2 Stunden gerührt. Nach üblicher Aufarbeitung erhält man das gewünschte Endprodukt.

Analog erhält man aus den entsprechenden imi-BOM- bzw. imi-DNP-Derivaten:

N-[5S-(BOC-Phe-His-amino)-2R,4S-dihydroxy-6-cyclohexylhexyl]-propansulfonsäureamid, F. 165-168°

N-[2S,4S-Dihydroxy-5S-(4-BOC-amino-piperidinocarbonyl-Phe-His-amino)-6-cyclohexyl-hexyl]-propansulfonsäureamid

N-[2S,4S-Dihydroxy-5S-(4-BOC-amino-piperidinocarbonyl-Phe-His-amino)-6-cyclohexyl-hexyl]-3-methylbutansäureamid, F. 120-130° (Zers.)

N-[2R,4S-Dihydroxy-5S-(4-BOC-amino-piperidinocarbonyl-Phe-His-amino)-6-cyclohexyl-hexyl]-3-methylbutansäureamid, F. 120-130° (Zers.)
N-[2S,4S-Dihydroxy-5S-(4-BOC-amino-piperidinocarbonyl-Phe-His-amino)-6-cyclohexyl-hexyl]-N'-isopropylharnstoff, F. 137-140°
N-[2R,4S-Dihydroxy-5S-(4-BOC-amino-piperidinocarbonyl-Phe-His-amino)-6-cyclohexyl-hexyl]-3-methylbutansäureamid, F. 137-140°
N-[2S,4S-Dihydroxy-5S-(4-BOC-amino-piperidinocarbonyl-Phe-His-amino)-6-cyclohexyl-hexyl]-N'-ethylharnstoff, F. 129° (Zers.)
N-[2R,4S-Dihydroxy-5S-(4-BOC-amino-piperidinocarbonyl-Phe-His-amino)-6-cyclohexyl-hexyl]-N'-ethylharnstoff, F. 143° (Zers.)
N-[2S,4S-Dihydroxy-5S-(2-benzyl-3-tert.-butyl-sulfonyl-propionyl-His-amino)-6-cyclohexyl-hexyl]-methansulfonsäureamid
N-[2S,4S-Dihydroxy-5S-(2-benzyl-3-tert.-butyl-sulfonyl-propionyl-His-amino)-6-cyclohexyl-hexyl]-propansulfonsäureamid
N-[2S,4S-Dihydroxy-5S-(2-benzyl-3-tert.-butyl-sulfonyl-propionyl-His-amino)-6-cyclohexyl-hexyl]-butansulfonsäureamid
N-[2S,4S-Dihydroxy-5S-(2-benzyl-heptanoyl-His-amino)-6-cyclohexyl-hexyl]-propansulfonsäureamid
N-[2S,4S-Dihydroxy-5S-(2-benzyl-4-oxo-5,5-dimethylhexanoyl-His-amino)-6-cyclohexyl-hexyl]-propansulfonsäureamid
N-[2S,4S-Dihydroxy-5S-(2-benzyl-3-tert.-butyl-sulfonylpropionyl-His-amino)-6-cyclohexyl-hexyl]-propansulfonsäureamid
N-[2S,4S-Dihydroxy-5S-(2-benzyl-3-morpholinocarbonyl-propionyl-His-amino)-6-cyclohexyl-hexyl]-propansulfonsäureamid
N-[25,4S-Dihydroxy-5S-(2-(1-naphthylmethyl)-3-morpholinocarbonyl-propionyl-His-amino)-6-cyclohexyl-hexyl]-propansulfonsäureamid
N-[25,4S-Dihydroxy-5S-(N-benzyl-N-isopentyl-carbamoyl-His-amino)-6-cyclohexyl-hexyl]-propansulfonsäureamid
N-[2S,4S-Dihydroxy-5S-(2-(1-naphthylmethyl)-3-(N-(3-dimethylaminopropyl)-carbamoyl-propionyl-His-amino)-6-cyclohexyl-hexyl]-propansulfonsäureamid
N-[2S,4S-Dihydroxy-5S-(2-benzyl-3-(4-BOC-aminopiperidinocarbonyl)-propionyl-His-amino)-6-cyclohexyl-hexyl]-propansulfonsäureamid
N-[2S,4S-Dihydroxy-5S-(2-(1-naphthylmethyl)-3-(4-BOC-amino-piperidinocarbonyl)-propionyl-His-amino)-6-cyclohexyl-hexyl]-propansulfonsäureamid
N-[2S,4S-Dihydroxy-5S-(CBZ-Phe-His-amino)-6-cyclohexyl-hexyl]-propansulfonsäureamid
N-[2S,4S-Dihydroxy-5S-(3,3-dimethylbutyryl-Phe-His-amino)-6-cyclohexyl-hexyl]-propansulfonsäureamid
N-[2S,4S-Dihydroxy-5S-(2-BOC-amino-2-methyl-propionyl-Phe-His-amino)-6-cyclohexyl-hexyl]-propansulfonsäureamid
N-[2S,4S-Dihydroxy-5S-(6-BOC-amino-hexanoyl-Phe-His-amino)-6-cyclohexyl-hexyl]-propansulfonsäureamid
N-[2S,4S-Dihydroxy-5S-(6-Methoxycarbonyl-hexanoyl-Phe-His-amino)-6-cyclohexyl-hexyl]-propansulfonsäureamid
N-[25,4S-Dihydroxy-5S-(N-(3-dimethylaminopropyl)-carbamoyl-Phe-His-amino)-6-cyclohexyl-hexyl]-propansulfonsäureamid
N-[2S,4S-Dihydroxy-5S-(N-(5-dimethylaminopentyl)-carbamoyl-Phe-His-amino)-6-cyclohexyl-hexyl]-propansulfonsäureamid
N-[2S,4S-Dihydroxy-5S-(BOC-Mal-His-amino)-6-cyclohexylhexyl]-propansulfonsäureamid
N-[2S,4S-Dihydroxy-5S-(CBZ-Mal-His-amino)-6-cyclohexylhexyl]-propansulfonsäureamid
N-[22,4S-Dihydroxy-5S-(3,3-dimethylbutyryl-Mal-His-amino)-6-cyclohexyl-hexyl]-propansulfonsäureamid
N-[2S,42-Dihydroxy-5S-(2-BOC-amino-2-methyl-propionyl-Mal-His-amino)-6-cyclohexyl-hexyl]-propansulfonsäureamid
N-[2S,4S-Dihydroxy-5S-(6-BOC-amino-hexanoyl-Mal-His-amino)-6-cyclohexyl-hexyl]-propansulfonsäureamid
N-[2S,4S-Dihydroxy-5S-(4-BOC-amino-piperidinocarbonyl-Mal-His-amino)-6-cyclohexyl-hexyl]-propansulfonsäureamid
N-[2S,4S-Dihydroxy-5S-(6-Methoxycarbonyl-hexanoyl-Mal-His-amino)-6-cyclohexyl-hexyl]-propansulfonsäureamid
N-[2S,4S-Dihydroxy-5S-(N-(3-dimethylaminopropyl)-carbamoyl-Mal-His-amino)-6-cyclohexyl-hexyl]-propansulfonsäureamid
N-[2S,4S-Dihydroxy-5S-(N-(5-dimethylaminopentyl)-carbamoyl-Mal-His-amino)-6-cyclohexyl-hexyl]-propansulfonsäureamid

N-[2S,4S-Dihydroxy-5S-(4-BOC-aminopiperidinocarbonyl-Mal-His-amino)-6-cyclohexyl-hexyl]-methansulfonsäureamid
N-[2S,4S-Dihydroxy-5S-(BOC-Phe-His-amino)-6-cyclohexyl-hexyl]-2-methylpropansulfonsäureamid
N-[2S,4S-Dihydroxy-5S-(BOC-Cal-His-amino)-6-cyclohexyl-hexyl]-2-methylpropansulfonsäureamid
N-[2S,4S-Dihydroxy-5S-(BOC-αNal-His-amino)-6-cyclohexyl-hexyl]-2-methylpropansulfonsäureamid
N-[2S,4S-Dihydroxy-5S-(BOC-2-Tia-His-amino)-6-cyclohexyl-hexyl]-2-methylpropansulfonsäureamid
N-[2S,4S-Dihydroxy-5S-(BOC-Trp-His-amino)-6-cyclohexyl-hexyl]-2-methylpropansulfonsäureamid
N-[2S,4S-Dihydroxy-5S-(BOC-Pro-Phe-His-amino)-6-cyclohexyl-hexyl]-2-methylpropansulfonsäureamid
N-[2S,4S-Dihydroxy-5S-(3,3-dimethylbutyryl-Pro-Phe-N-Me-His-amino)-6-cyclohexyl-hexyl]-2-methylpropansulfonsäureamid
N-[2S,4S-Dihydroxy-5S-(4-CBZ-amino-piperidinocarbonyl-Phe-His-amino)-6-cyclohexyl-hexyl]-propansulfonsäureamid.

N-[2S,4S-Dihydroxy-5S-(2-benzyl-3-tert.-butylsulfonyl-propionyl-His-amino)-6-cyclohexyl-hexyl]-methansulfonsäureamid
N-[2S,4S-Dihydroxy-5S-(2-benzyl-3-tert.butylsulfonyl-propionyl-His-amino)-6-cyclohexyl-hexyl]-butansulfonsäureamid
N-[2S,4S-Dihydroxy-5S-(2-benzyl-3-tert.-butylsulfonyl-propionyl-His-amino)-6-cyclohexyl-hexyl]-2-methylpropansulfonsäureamid
N-[2S,4S-Dihydroxy-5S-(2-benzyl-3-tert.-butylsulfonyl-propionyl-His-amino)-6-cyclohexyl-hexyl]-phenylmethansulfonsäureamid
N-[2S,4S-Dihydroxy-5S-(2-benzyl-3-tert.-butylsulfonyl-propionyl-His-amino)-6-cyclohexyl-hexyl]-cyclohexylmethan-sulfonsäureamid
N-[2S,4S-Dihydroxy-5S-(2-benzyl-3-tert.-butylsulfonyl-propionyl-His-amino)-6-cyclohexyl-hexyl]-N'-propylharnstoff
N-[2S,4S-Dihydroxy-5S-(2-benzyl-3-tert.-butylsulfonyl-propionyl-His-amino)-6-cyclohexyl-hexyl]-N'-butylharnstoff
N-[2S,4S-Dihydroxy-5S-(2-benzyl-3-tert.-butylsulfonyl-propionyl-His-amino)-6-cyclohexyl-hexyl]-N'-propylthioharnstoff
N-[2S,4S-Dihydroxy-5S-(2-benzyl-3-tert.-butylsulfonyl-propionyl-His-amino)-6-cyclohexyl-hexyl]-N'-(p-aminophenyl)-harnstoff
N-[2S,4S-Dihydroxy-5S-(2-benzyl-3-tert.-butylsulfonyl-propionyl-His-amino)-6-cyclohexyl-hexyl]-4-methylpentansäureamid.

Beispiel 2

Eine Lösung von 0,01 mol N-[2S,4S-Dihydroxy-5S-(H-βAla-amino)-6-cyclohexyl-hexyl]-propansulfonsäureamid [erhältlich durch Kondensation von BOC-βAla-OH mit N-(2S,4S-Dihydroxy-5S-amino-6-cyclohexyl-hexyl)-propansulfonsäureamid zu N-[2S,4S-Dihydroxy-5S-(BOC-βAla-amino)-6-cyclohexyl-hexyl]-propansulfonsäureamid und Abspaltung der BOC-Gruppe mit 4 n HCl in Dioxan] in 60 ml Dichlormethan wird mit 0,01 mol N-Methylmorpholin versetzt. Unter Rühren gibt man 0,01 mol 4-Dimethylamino-piperidinocarbonyl-Phe-OH, 1,35 g HOBt und eine Lösung von 2,06 g DCCI in 50 ml Dichlormethan hinzu, rührt 14 Std. bei 2-6°, filtriert den ausgeschiedenen Dicyclohexylharnstoff ab und dampft das Filtrat ein. Nach üblicher Aufarbeitung erhält man N-[2S,4S-Dihydroxy-5S-(4-dimethylamino-piperidinocarbonyl-Phe-βAla-amino)-6-cyclohexyl-hexyl]-propansulfonsäureamid.

Analog erhält man mit 4-BOC-amino-piperidinocarbonyl-Phe-OH das N-[2S,4S-Dihydroxy-5S-(4-BOC-amino-piperidinocarbonyl-Phe-βAla-amino)-6-cyclohexyl-hexyl]-propansulfonsäureamid als farblosen Schaum.

Analog erhält man:
N-[2S,4S-Dihydroxy-5S-(4-BOC-amino-piperidinocarbonyl-Phe-βAla-amino)-6-cyclohexyl-hexyl]-2-propansulfonsäureamid, farbl. Schaum
N-[2S,4S-Dihydroxy-5S-(4-BOC-amino-piperidinocarbonyl-Phe-2-Tia-amino)-6-cyclohexyl-hexyl]-propansulfonsäureamid
N-[2S,4S-Dihydroxy-5S-(N-(5-dimethylaminopentyl)-carbamoyl-Phe-βAla-amino)-6-cyclohexyl-hexyl]-propansulfonsäureamid
N-[2S,4S-Dihydroxy-5S-(N-(5-dimethylaminopentyl)-carbamoyl-Phe-2-Tia-amino)-6-cyclohexyl-hexyl]-propansulfonsäureamid

Beispiel 3

Analog Beispiel 2 erhält man aus BOC-Phe-Gly-OH und N-(2S,4S-Dihydroxy-5S-amino-6-cyclohexyl-hexyl)-propansulfonsäureamid das N-(2S,4S-Dihydroxy-5S-(BOC-Phe-Gly-amino)-6-cyclohexyl-hexyl)-propansulfonsäureamid.

Analog erhält man
N-(2S,4S-Dihydroxy-5S-(BOC-Phe-Abu-amino)-6-cyclohexyl-hexyl)-propansulfonsäureamid
N-(2S,4S-Dihydroxy-5S-(BOC-Phe-Ala-amino)-6-cyclohexyl-hexyl)-propansulfonsäureamid
N-(2S,4S-Dihydroxy-5S-(BOC-Phe-βAla-amino)-6-cyclohexyl-hexyl)-propansulfonsäureamid
N-(2S,4S-Dihydroxy-5S-(BOC-Phe-Asn-amino)-6-cyclohexyl-hexyl)-propansulfonsäureamid
N-(2S,4S-Dihydroxy-5S-(BOC-Phe-Gln-amino)-6-cyclohexyl-hexyl)-propansulfonsäureamid
N-(2S,4S-Dihydroxy-5S-(BOC-Phe-Nle-amino)-6-cyclohexyl-hexyl)-propansulfonsäureamid
N-2R,4S-Dihydroxy-5S-(BOC-Phe-Ser-amino)-6-cyclohexyl-hexyl)-propansulfonsäureamid
N-2R,4S-Dihydroxy-5S-(BOC-Phe-amino)-6-cyclohexyl-hexyl)-propansulfonsäureamid
N-(2S,4S-Dihydroxy-5S-(BOC-Phe-3-Pya-amino)-6-cyclohexyl-hexyl)-propansulfonsäureamid
N-(2S,4S-Dihydroxy-5S-(BOC-Phe-2-Tia-amino)-6-cyclohexyl-hexyl)-propansulfonsäureamid

Beispiel 4

Analog Beispiel 2 erhält man aus 4-BOC-aminopiperidinocarbonyl-Phe-OH und N-[2S,4S-Dihydroxy-5S-(H-Gly-amino)-7-methyl-octyl]-ethansulfonsäureamid das N-[2S,4S-Dihydroxy-5S-(4-BOC-aminopiperidinocarbonyl-Phe-Gly-amino)-7-methyl-octyl]-ethansulfonsäureamid.

Beispiel 5

Eine Lösung von 1 g N-[2S,4S-Dihydroxy-5S-(4-BOC-aminopiperidinocarbonyl-Phe-His-amino)-6-cyclohexyl-hexyl]-propansulfonsäureamid in 20 ml 4 n HCl in Dioxan wird 30 Min. bei 20° gerührt und dann eingedampft. Man erhält N-[2S,4S-Dihydroxy-5S-(4-amino-piperidinocarbonyl-Phe-His-amino)-6-cyclohexyl-hexyl]-propansulfonsäureamid, Dihydrochlorid.

Analog erhält man durch Spaltung der entsprechenden BOC-Derivate:
N-[2S,4S-Dihydroxy-5S-(2-benzyl-3-(4-aminopiperidinocarbonyl)-propionyl-His-amino)-6-cyclohexyl-hexyl]-propansulfonsäureamid
N-[2S,4S-Dihydroxy-5S-(2-(1-naphthylmethyl)-3-(4-aminopiperidinocarbonyl)-propionyl-His-amino)-6-cyclohexylhexyl]-propansulfonsäureamid
N-[2S,4S-Dihydroxy-5S-(4-aminopiperidinocarbonyl-Phe-βAla-amino)-6-cyclohexyl-hexyl]-propansulfonsäureamid
N-[2S,4S-Dihydroxy-5S-(4-aminopiperidinocarbonyl-Phe-2-Tia-amino)-6-cyclohexyl-hexyl]-propansulfonsäureamid
N-[2S,4S-Dihydroxy-5S-(4-aminopiperidinocarbonyl-Phe-His-amino)-6-cyclohexyl-hexyl]-propansulfonsäureamid
N-[2S,4S-Dihydroxy-5S-(4-aminopiperidinocarbonyl-Mal-His-amino)-6-cyclohexyl-hexyl]-propansulfonsäureamid
N-[2S,4S-Dihydroxy-5S-(2-amino-2-methyl-propionyl-Phe-His-amino)-6-cyclohexyl-hexyl]-propansulfonsäureamid
N-[2S,4S-Dihydroxy-5S-(2-amino-2-methyl-propionyl-Mal-His-amino)-6-cyclohexyl-hexyl]-propansulfonsäureamid
N-[2S,4S-Dihydroxy-5S-(6-aminohexanoyl-Phe-His-amino)-6-cyclohexyl-hexyl]-propansulfonsäureamid
N-[2S,4S-Dihydroxy-5S-(6-aminohexanoyl-Mal-His-amino)-6-cyclohexyl-hexyl]-propansulfonsäureamid
N-[2S,4S-Dihydroxy-5S-(4-aminopiperidinocarbonyl-Phe-βAla-amino)-6-cyclohexyl-hexyl]-2-propansulfonsäureamid
H-[2S,4S-Dihydroxy-5S-(4-aminopiperidinocarbonyl-Phe-His-amino)-6-cyclohexyl-hexyl]-3-methylbutansäureamid
N-[2S,4S-Dihydroxy-5S-(4-aminopiperidinocarbonyl-Phe-His-amino)-6-cyclohexyl-hexyl]-N'-isopropylharnstoff
N-[2S,4S-Dihydroxy-5S-(4-aminopiperidinocarbonyl-Phe-His-amino)-6-cyclohexyl-hexyl]-methansulfonsäureamid
N-[2S,4S-Dihydroxy-5S-(4-aminopiperidinocarbonyl-Mal-His-amino)-6-cyclohexyl-hexyl]-methansulfonsäureamid.

Beispiel 6

Durch Hydrogenolyse (an 10%ig. Pd-C in Ethanol bei 20°) erhält man aus N-[2S,4S-Dihydroxy-5S-(4-CBZ-aminopiperidinocarbonyl-Phe-His-amino)-6-cyclohexyl-hexyl]-propansulfonsäureamid das N-[2S,4S-Dihydroxy-5S-(4-amino-piperidinocarbonyl-Phe-His-amino)-6-cyclohexyl-hexyl]-propansulfonsäureamid.

Beispiel 7

Analog Beispiel 1 erhält man aus den entsprechenden imi-BOM-His-Derivaten:

N-[2R,4S-Dihydroxy-5S-(3-BOC-amino-3-methylbutyryl-Phe-His-amino)-6-cyclohexyl-hexyl]-propansulfonsäureamid
N-[2R,4S-Dihydroxy-5S-(3-BOC-amino-3-methylbutyryl-Mal-His-amino)-6-cyclohexyl-hexyl]-propansulfonsäureamid
N-[2R,4S-Dihydroxy-5S-(3-BOC-amino-3-methylbutyryl-Phe-N-Me-His-amino)-6-cyclohexyl-hexyl]-propansulfonsäureamid
N-[2R,4S-Dihydroxy-5S-(3-BOC-amino-3-methylbutyryl-Phe-His amino)-6-cyclohexyl-hexyl]-1-methylethansulfonsäureamid
N-[2R,4S-Dihydroxy-5S-(3-BOC-amino-3-methylbutyryl-Mal-His-amino)-6-cyclohexyl-hexyl]-1-methylethansulfonsäureamid
N-[2R,4S-Dihydroxy-5S-(3-BOC-amino-3-methylbutyryl-Phe-N-Me-His-amino)-6-cyclohexyl-hexyl]-1-methylethansulfonsäureamid
N-[2R,4S-Dihydroxy-5S-(3-BOC-amino-3-methylbutyryl-Phe-His-amino)-6-cyclohexyl-hexyl]-cyclohexan-sulfonsäureamid
N-[2R,4S-Dihydroxy-5S-(3-BOC-amino-3-methylbutyryl-Phe-His-amino)-6-cyclohexyl-hexyl)-N'-ethyl-harnstoff
N-[2R,4S-Dihydroxy-5S-(3-BOC-amino-3-methylbutyryl-Phe-N-Me-His-amino)-6-cyclohexyl-hexyl]-N'-isopropyl-harnstoff
N-[2R,4S-Dihydroxy-5S-(4-BOC-amino-piperidinocarbonyl-Phe-His-amino)-6-cyclohexyl-hexyl]-1-methylethansulfonsäureamid
N-[2R,4S-Dihydroxy-5S-(4-BOC-amino-piperidinocarbonyl-Mal-His-amino)-6-cyclohexyl-hexyl]-1-methylethansulfonsäureamid
N-[2R,4S-Dihydroxy-5S-(4-BOC-amino-piperidinocarbonyl-Phe-His-amino)-6-cyclohexyl-hexyl]-cyclohexansulfonsäureamid
N-[2R,4S-Dihydroxy-5S-(4-BOC-amino-piperidinocarbonyl-Mal-His-amino)-6-cyclohexyl-hexyl]-cyclohexansulfonsäureamid
N-[2R,4S-Dihydroxy-5S-(4-BOC-amino-piperidinocarbonyl-Phe-His-amino)-6-cyclohexyl-hexyl]-N'-ethyl-harnstoff
N-[2R,4S-Dihydroxy-5S-(4-BOC-amino-piperidinocarbonyl-Mal-His-amino)-6-cyclohexyl-hexyl]-N'-ethyl-harnstoff
N-[2R,4S-Dihydroxy-5S-(4-BOC-amino-piperidinocarbonyl-Phe-His-amino)-6-cyclohexyl-hexyl]-N'-isopropyl-harnstoff
N-[2R,4S-Dihydroxy-5S-(4-BOC-amino-piperidinocarbonyl-Mal-His-amino)-6-cyclohexyl-hexyl]-N'-isopropyl-harnstoff
N-[2R,4S-Dihydroxy-5S-(4-BOC-amino-piperidinocarbonyl-Phe-His-amino)-6-cyclohexyl-hexyl]-N'-phenyl-harnstoff
N-[2R,4S-Dihydroxy-5S-(4-BOC-amino-piperidinocarbonyl-Mal-His-amino)-6-cyclohexyl-hexyl]-N'-phenyl-harnstoff
N-[2R,4S-Dihydroxy-5S-(4-BOC-amino-piperidinocarbonyl-Phe-His-amino)-6-cyclohexyl-hexyl]-3-methylbutyramid
N-[2R,4S-Dihydroxy-5S-(4-BOC-amino-piperidinocarbonyl-Mal-His-amino)-6-cyclohexyl-hexyl]-3-methylbutyramid
N-[2R,4S-Dihydroxy-5S-(2R-benzyl-4-(4-BoC-amino-piperidinocarbonyl)-propionyl-His-amino)-6-cyclohexyl-hexyl]-propansulfonsäureamid
N-[2R,4S-Dihydroxy-5S-(2S-benzyl-4-(4-BOC-amino-piperidinocarbonyl)-propionyl-His-amino)-6-cyclohexyl-hexyl]-propansulfonsäureamid
N-[2R,4S-Dihydroxy-5S-(2R-(1-naphthylmethyl)-3-(4-BOC-amino-piperidinocarbonyl)-propionyl-His-amino)-6-cyclohexyl-hexyl]-propansulfonsäureamid
N-[2R,4S-Dihydroxy-5S-(2S-(1-naphthylmethyl)-3-(4-BOC-amino-piperidinocarbonyl)-propionyl-His-amino)-6-cyclohexyl-hexyl]-propansulfonsäureamid
N-[2R,4S-Dihydroxy-5S-(2R-benzyl-5-N-BOC-N-methyl-aminopentanoyl-His-amino)-6-cyclohexyl-hexyl]-

propansulfonsäureamid
N-[2R,4S-Dihydroxy-5S-(2S-benzyl-5-N-BOC-N-methyl-aminopentanoyl-His-amino)-6-cyclohexyl-hexyl]-propansulfonsäureamid
N-[2R,4S-Dihydroxy-5S-(2R-benzyl-5-N-BOC-N-methyl-aminopentanoyl-His-amino)-6-cyclohexyl-hexyl]-1-methylethansulfonsäureamid
N-[2R,4S-Dihydroxy-5S-(25-benzyl-5-N-BOC-N-methyl-aminopentanoyl-His-amino)-6-cyclohexyl-hexyl]-1-methylethansulfonsäureamid
N-[2R,4S-Dihydroxy-5S-(2R-benzyl-5-N-BOC-N-methyl-aminopentanoyl-His-amino)-6-cyclohexyl-hexyl]-N'-ethyl-harnstoff
N-[2R,4S-Dihydroxy-5S-(2S-benzyl-5-N-BOC-N-methyl-aminopentanoyl-His-amino)-6-cyclohexyl-hexyl]-N'-ethyl-harnstoff
N-[2R,4S-Dihydroxy-5S-(2R-benzyl-3-tert.-butylsulfonyl-propionyl-His-amino)-6-cyclohexyl-hexyl]-1-methyl-ethansulfonsäureamid
N-[2R,4S-Dihydroxy-5S-(2S-benzyl-3-tert.-butylsulfonyl-propionyl-His-amino)-6-cyclohexyl-hexyl]-1-methyl-ethansulfonsäureamid
N-[2R,4S-Dihydroxy-5S-(2R-benzyl-3-tert.-butylsulfonylpropionyl-His-amino)-6-cyclohexyl-hexyl]-cyclohexansulfonsäureamid
N-[2R,4S-Dihydroxy-5S-(2S-benzyl-3-tert.-butylsulfonyl-propionyl-His-amino)-6-cyclohexyl-hexyl]-cyclohexansulfonsäureamid
N-[2R,4S-Dihydroxy-5S-(2R-benzyl-3-tert.-butylsulfonyl-propionyl-His-amino)-6-cyclohexyl-hexyl]-N'-ethylharnstoff
N-[2R,4S-Dihydroxy-5S-(2S-benzyl-3-tert.-butylsulfonyl-propionyl-His-amino)-6-cyclohexyl-hexyl]-N'-ethylharnstoff
N-[2R,4S-Dihydroxy-5S-(2R-benzyl-3-tert.-butylsulfonyl-propionyl-His-amino)-6-cyclohexyl-hexyl)-N'-isopropylharnstoff
N-[2R,4S-Dihydroxy-5S-(2S-benzyl-3-tert.-butylsulfonyl-propionyl-His-amino)-6-cyclohexyl-hexyl)-N'-isopropylharnstoff
N-[2R,4S-Dihydroxy-5S-(2R-benzyl-heptanoyl-His-amino)-6-cyclohexyl-hexyl]-propansulfonsäureamid
N-[2R,4S-Dihydroxy-5S-(2S-benzyl-heptanoyl-His-amino)-6-cyclohexyl-hexyl]-propansulfonsäureamid
N-[2R,4S-Dihydroxy-5S-(2R-(1-naphthylmethyl)-3-morpholinocarbonyl-propionyl-His-amino)-6-cyclohexyl-hexyl]-propansulfonsäureamid
N-[2R,4S-Dihydroxy-5S-(2S-(1-naphthylmethyl)-3-morpholinocarbonyl-propionyl-His-amino)-6-cyclohexyl-hexyl]-propansulfonsäureamid
N-[2R,4S-Dihydroxy-5S-(2R-benzyl-3-tert.-butylthio-propionyl-His-amino)-6-cyclohexyl-hexyl]-propansulfonsäureamid
N-[2R,4S-Dihydroxy-5S-(2S-benzyl-3-tert.-butylthiopropionyl-His-amino)-6-cyclohexyl-hexyl]-propansulfonsäureamid
N-[2R,4S-Dihydroxy-5S-(2R-benzyl-3-tert.-butylsulfinylpropionyl-His-amino)-6-cyclohexyl-hexyl]-propansulfonsäureamid
N-[2R,4S-Dihydroxy-5S-(2S-benzyl-3-tert.-butylsulfinyl-propionyl-His-amino)-6-cyclohexyl-hexyl]-propansulfonsäureamid
N-[2R,4S-Dihydroxy-5S-(2R-benzyl-4-oxo-5,5-dimethylhexanoyl-His-amino)-6-cyclohexyl-hexyl]-propansulfonsäureamid
N-[2R,4S-Dihydroxy-5S-(2S-benzyl-4-oxo-5,5-dimethylhexanoyl-His-amino)-6-cyclohexyl-hexyl]-propansulfonsäureamid
N-[2R,4S-Dihydroxy-5S-(2R-benzyl-4-hydroxy-5,5-dimethylhexanoyl-His-amino)-6-cyclohexyl-hexyl]-propansulfonsäureamid
N-[2R,4S-Dihydroxy-5S-(2S-benzyl-4-hydroxy-5,5-dimethylhexanoyl-His-amino)-6-cyclohexyl-hexyl]-propansulfonsäureamid
N-[2R,4S-Dihydroxy-5S-(4-dimethylamino-piperidinocarbonyl-Phe-His-amino)-6-cyclohexyl-hexyl]-propansulfonsäureamid
N-[2R,4S-Dihydroxy-5S-(4-dimethylamino-piperidinocarbonyl-Mal-His-amino)-6-cyclohexyl-hexyl]-propansulfonsäureamid
N-[2R,4S-Dihydroxy-5S-(N-(5-dimethylaminopentyl)-carbonyl-Phe-His-amino)-6-cyclohexyl-hexyl]-propansulfonsäureamid
N-[2R,4S-Dihydroxy-5S-(N-(5-dimethylaminopentyl)-carbonyl-Mal-His-amino)-6-cyclohexyl-hexyl]-propansulfonsäureamid.

Beispiel 8

Analog Beispiel 5 erhält man aus den entsprechenden BOC-Derivaten:
N-[2R,4S-Dihydroxy-5S-(4-amino-piperidinocarbonyl-Phe-His-amino)-6-cyclohexyl-hexyl]-propansulfonsäureamid, Hydrochlorid
N-[2R,4S-Dihydroxy-5S-(4-amino-piperidinocarbonyl-Mal-His-amino)-6-cyclohexyl-hexyl]-propansulfonsäureamid
N-[2R,4S-Dihydroxy-5S-(3-amino-3-methylbutyryl-Phe-His-amino)-6-cyclohexyl-hexyl]-propansulfonsäureamid
N-[2R,4S-Dihydroxy-5S-(3-amino-3-methylbutyryl-Mal-His-amino)-6-cyclohexyl-hexyl]-propansulfonsäureamid
N-[2R,4S-Dihydroxy-5S-(3-amino-3-methylbutyryl-Phe-N-Me-His-amino)-6-cyclohexyl-hexyl]-propansulfonsäureamid
N-[2R,4S-Dihydroxy-5S-(3-amino-3-methylbutyryl-Phe-His-amino)-6-cyclohexyl-hexyl]-1-methyl-ethansulfonsäureamid
N-[2R,4S-Dihydroxy-5S-(3-amino-3-methylbutyryl-Mal-His-amino)-6-cyclohexyl-hexyl]-1-methyl-ethansulfonsäureamid
N-[2R,4S-Dihydroxy-5S-(3-amino-3-methylbutyryl-Phe-N-Me-His-amino)-6-cyclohexyl-hexyl]-1-methyl-ethansulfonsäureamid
N-[2R,4S-Dihydroxy-5S-(3-amino-3-methylbutyryl-Phe-His-amino)-6-cyclohexyl-hexyl]-cyclohexansulfonsäureamid
N-[2R,4S-Dihydroxy-5S-(3-amino-3-methylbutyryl-Phe-Mal-amino)-6-cyclohexyl-hexyl]-cyclohexansulfonsäureamid
N-[2R,4S-Dihydroxy-5S-(3-amino-3-methylbutyryl-Phe-His-amino)-6-cyclohexyl-hexyl]-N'-ethyl-harnstoff
N-[2R,4S-Dihydroxy-5S-(3-amino-3-methylbutyryl-Phe-Mal-amino)-6-cyclohexyl-hexyl]-N'-ethyl-harnstoff
N-[2R,4S-Dihydroxy-5S-(3-amino-3-methylbutyryl-Phe-N-Me-His-amino)-6-cyclohexyl-hexyl]-N'-isopropylharnstoff
N-[2R,4S-Dihydroxy-5S-(4-amino-piperidinocarbonyl-Phe-His-amino)-6-cyclohexyl-hexyl]-1-methylethansulfonsäureamid
N-[2R,4S-Dihydroxy-5S-(4-amino-piperidinocarbonyl-Mal-His-amino)-6-cyclohexyl-hexyl]-1-methylethansulfonsäureamid
N-[2R,4S-Dihydroxy-5S-(4-amino-piperidinocarbonyl-Phe-His-amino)-6-cyclohexyl-hexyl]-cyclohexansulfonsäureamid
N-[2R,4S-Dihydroxy-5S-(4-amino-piperidinocarbonyl-Mal-His-amino)-6-cyclohexyl-hexyl]-cyclohexansulfonsäureamid
N-[2R,4S-Dihydroxy-5S-(4-amino-piperidinocarbonyl-Phe-His-amino)-6-cyclohexyl-hexyl]-N'-ethyl-harnstoff
N-[2R,4S-Dihydroxy-5S-(4-amino-piperidinocarbonyl-Mal-His-amino)-6-cyclohexyl-hexyl]-N'-ethyl-harnstoff
N-[2R,4S-Dihydroxy-5S-(4-amino-piperidinocarbonyl-Phe-His-amino)-6-cyclohexyl-hexyl]-N'-isopropyl-harnstoff
N-[2R,4S-Dihydroxy-5S-(4-amino-piperidinocarbonyl-Mal-His-amino)-6-cyclohexyl-hexyl]-N'-isopropyl-harnstoff
N-[2R,4S-Dihydroxy-5S-(4-amino-piperidinocarbonyl-Phe-His-amino)-6-cyclohexyl-hexyl]-N'-phenyl-harnstoff
N-[2R,4S-Dihydroxy-5S-(4-amino-piperidinocarbonyl-Mal-His-amino)-6-cyclohexyl-hexyl]-N'-phenyl-harnstoff
N-[2R,4S-Dihydroxy-5S-(4-amino-piperidinocarbonyl-Phe-His-amino)-6-cyclohexyl-hexyl]-3-methylbutyramid
N-[2R,4S-Dihydroxy-5S-(4-amino-piperidinocarbonyl-Mal-His-amino)-6-cyclohexyl-hexyl]-3-methylbutyramid
N-[2R,4S-Dihydroxy-5S-(2R-benzyl-3-(4-amino-piperidinocarbonyl)-propionyl-His-amino)-6-cyclohexyl-hexyl]-propansulfonsäureamid
N-[2R,4S-Dihydroxy-5S-(4-aminopiperidinocarbonyl-Phe-βAla-amino)-6-cyclohexyl-hexyl]-2-propansulfonsäureamid
N-[2R,4S-Dihydroxy-5S-(2S-benzyl-3-(4-amino-piperidinocarbonyl)-propionyl-His-amino)-6-cyclohexyl-hexyl]-propansulfonsäureamid
N-[2R,4S-Dihydroxy-5S-(2R-(1-naphthylmethyl)-3-(4-amino-piperidinocarbonyl)-propionyl-His-amino)-6-cyclohexyl-hexyl]-propansulfonsäureamid
N-[2R,4S-Dihydroxy-5S-(2S-(1-naphthylmethyl)-3-(4-amino-piperidinocarbonyl)-propionyl-His-amino)-6-cyclohexyl-hexyl]-propansulfonsäureamid
N-[2R,4S-Dihydroxy-5S-(2R-benzyl-5-N-methyl-amino-pentanoyl-His-amino)-6-cyclohexyl-hexyl]-propansulfonsäureamid
N-[2R,4S-Dihydroxy-5S-(2S-benzyl-5N-methyl-amino-pentanoyl-His-amino)-6-cyclohexyl-hexyl]-

propansulfonsäureamid
N-[2R,4S-Dihydroxy-5S-(2R-benzyl-5-N-methyl-amino-pentanoyl-His-amino)-6-cyclohexyl-hexyl]-1-methylethansulfonsäureamid
N-[2R,4S-Dihydroxy-5S-(2S-benzyl-5-N-methyl-amino-pentanoyl-His-amino)-6-cyclohexyl-hexyl]-1-methylethansulfonsäureamid
N-[2R,4S-Dihydroxy-5S-(2R-benzyl-5-N-methyl-amino-pentanoyl-His-amino)-6-cyclohexyl-hexyl]-N'-ethylharnstoff
N-[2R,4S-Dihydroxy-5S-(2S-benzyl-5-N-methyl-amino-pentanoyl-His-amino)-6-cyclohexyl-hexyl]-N'-ethylharnstoff.

Beispiel 9

Ein Gemisch von 730 mg N-[2R,4S-Dihydroxy-5S-(4-aminopiperidino-carbonyl-Phe-His-amino)-6-cyclohexyl-hexyl]-propansulfonsäureamid, 90 mg S-Methylisothioharnstoff, 5 ml Ethanol und 5 ml Wasser wird 2 Std. bei 50° gerührt. Nach üblicher Aufarbeitung erhält man N-[2R,4S-Dihydroxy-5S-(4-guanidinopiperidinocarbonyl-Phe-His-amino)-6-cyclohexyl-hexyl]-propansulfonsäureamid.

Analog erhält man N-[2R,4S-Dihydroxy-5S-(4-guanidinopiperidinocarbonyl-Mal-His-amino)-6-cyclohexyl-hexyl]-propansulfonsäureamid.

Beispiel 10

Ein Gemisch von 730 mg N-[2R,4S-Dihydroxy-5S-(4-aminopiperidino-carbonyl-Phe-His-amino)-6-cyclohexyl-hexyl]-propansulfonsäureamid, 115 mg Trimethylsilylisocyanat und 10 ml THF wird 16 Std. bei 20° gerührt. Danach rührt man in Wasser ein, arbeitet wie üblich auf und erhält N-[2R,4S-Dihydroxy-5S-(4-ureido-piperidinocarbonyl-Phe-His-amino)-6-cyclohexyl-hexyl]-propansulfonsäureamid.

Analog erhält man N-[2R,4S-Dihydroxy-5S-(4-ureido-piperidinocarbonyl-Mal-His-amino)-6-cyclohexyl-hexyl]-propansulfonsäureamid,
sowie mit Ethylisocyanat:
N-[2R,4S-Dihydroxy-5S-(4-N'-ethylureido-piperidinocarbonyl-Phe-His-amino)-6-cyclohexyl-hexyl]-propansulfonsäureamid:
N-[2R,4S-Dihydroxy-5S-(4-N'-ethylureido-piperidinocarbonyl-Mal-His-amino)-6-cyclohexyl-hexyl]-propansulfonsäureamid.

Beispiel 11

a) 0,48 g (0,53 mMol) 2,2-Dimethyl-4S-[2-cyclohexyl-1S-(BOC-Phe-His(DNP)-amino)-ethyl]-6R-(3-isopropylureido)-methyl-1,3-dioxan [erhältlich durch Umsetzung von 3-[(4S,5S)-3-BOC-4-cyclohexylmethyl-2,2-dimethyl-oxazolidinyl-(5)]-(2R,S)-2-trimethylsilyloxy)-propylamin (siehe Beispiel 1) mit Isopropylisocyanat in THF in Gegenwart von Triethylamin bei 0° zu N-[3-{(4S,5S)-3-BOC-4-cyclohexyl-methyl-2,2-dimethyloxazolidinyl-(5)}-(2R,S)-trimethylsilyloxypropyl]-N'-isopropylharnstoff, chromatographische Trennung der Diastereomeren; Abspaltung der Trimethylsilylgruppe aus dem 2R-Epimeren durch Umsetzung mit Tetrabutylammoniumfluorid bei 0°, dieses Produkt wird in Essigsäureethylester mit HCl-gesättigtem Essigsäureethylester bei 0° 48 Stunden gerührt, man gibt Wasser hinzu und extrahiert mit 32%iger NaOH, die organische Phase wird aufgearbeitet und nach Reinigung durch Chromatographie an Kieselgel erhält man 2,2-Dimethyl-4S-[2-cyclohexyl-1S-aminoethyl]-6R-(3-isopropylureido)-methyl-1,3-dioxan; Kondensation mit BOC-Phe-His(DNP)-OH in DMF in Gegenwart von HOBt, NMM und DAPECl] werden in 5 ml DMF mit 0,2 ml 5%iger $NaHCO_3$-Lösung versetzt, anschließend werden 0,14 ml (2mMo1) 2-Mercaptoethanol zugefügt. Nach 12 Stunden werden 50 ml $H_2O$
zugesetzt, der sich bildende Niederschlag in $CH_2Cl_2$ aufgenommen. Der kristalline Rückstand wird an Kieselgel gereinigt und man erhält 2,2-Dimethyl-4S-[2-cyclohexyl-1S-(BOC-Phe-His-amino)-ethyl]-6R-(3-isopropylureido)methyl-1,3-dioxan, F. 134-140° (Zers.).

Analog erhält man:
4S-[2-cyclohexyl-1S-{(4-BOC-amino-piperidinocarbonyl)-Phe-His-amino}-ethyl]-2-methyl-6R-(3-methylbutyrylaminomethyl)-1,3-dioxan

b) Durch Abspaltung der BOC-Gruppe nach bekannter Art und Weise erhält man
2,2-Dimethyl-4S-[2-cyclohexyl-1S-(Phe-His-amino)-ethyl]-6R-(3-isopropylureido)-methyl-1,3-dioxan und
4S-[2-cyclohexyl-1S-{(4-aminopiperidinocarbonyl)-Phe-His-amino}-ethyl]-2-methyl-6R-(3-methylbutyrylaminomethyl)-1,3-dioxan.

Die nachfolgenden Beispiele betreffen pharmazeutische Zubereitungen.

Beispiel A: Tabletten

Ein Gemisch von 1 kg N-[2R,4S-Dihydroxy-5S-(4-aminopiperidino-carbonyl-Phe-His-amino)-6-cyclohexyl-hexyl]-3-methylbutyramid, 4 kg Lactose, 1,2 kg Maisstärke, 200 g Talk und 100 g Magnesiumstearat wird in üblicher Weise zu Tabletten verpreßt, derart daß jede Tablette 100 mg Wirkstoff enthält.

Beispiel B: Dragees

Analog Beispiel A werden Tabletten gepreßt, die anschließend in üblicher Weise mit einem Überzug aus Saccharose, Maisstärke, Talk, Tragant und Farbstoff überzogen werden.

Beispiel C: Kapseln

500 g N-[25,4S-Dihydroxy-5S-(4-amino-piperidinocarbonyl-Phe-βAla-amino)-6-cyclohexyl-hexyl]-propansulfonsäureamid-hydrochlorid werden in üblicher Weise in Hartgelatinekapseln gefüllt, so daß jede Kapsel 500 mg Wirkstoff enthält.

Beispiel D: Injektionsgläser

Eine Lösung von 100 g N-[2S,4S-Dihydroxy-5S-(4-aminopiperidinocarbonyl-Phe-His-amino)-6-cyclohexyl-hexyl]-propansulfonsäureamid-dihydrochlorid in 4 l zweifach destilliertem Wasser wird mit 2n Salzsäure auf pH 6,5 eingestellt, steril filtriert und in Injektionsgläser abgefüllt. Man lyophilisiert unter sterilen Bedingungen und verschließt steril. Jedes Injektionsglas enthält 50 mg Wirkstoff.

Beispiel E: Suppositorien

Man schmilzt ein Gemisch von 50 g N-[2S,4S-Dihydroxy-5S-(2-benzyl-3-tert.-butylsulfonyl-propionyl-His-amino)-6-cyclohexyl-hexyl]-methansulfonsäureamid-hydrochlorid mit 10 g Sojalecithin und 140 g Kakaobutter, gießt in Formen und läßt erkalten. Jedes Suppositorium enthält 250 mg Wirkstoff.

**Patentansprüche**

**1.** Peptid-Analoga der Formel I

$R^1$-Z-$NR^2$-$CHR^3$-$CR^4$-$CH_2$-$CR^5R^6$-Y   I

worin

| | |
|---|---|
| $R^1$ | H, $R^7$-O-$C_mH_{2m}$-CO- $R^7$-$C_mH_{2m}$-O-CO-, $R^7$-CmH2m-CO-, $R^7$-$SO_2$-, $R^8R^9$N-$C_mH_{2m}$-CO-, $R^{10}$-NH-C(=NH)-NH-$C_mH_{2m}$-CO-, $R^8$OOC-$C_mH_{2m}$-CO-, $R^8O_3$S-$C_mH_{2m}$-CO- oder $R^{11}$-$C_mH_{2m}$-$(T)_s$-$(V)_y$-$C_nH_{2n}$-L($R^7$-$C_pH_{2p}$)-$C_rH_{2r}$-CO-, |
| Z | 0 bis 4 peptidartig miteinander verbundene Aminosäurereste ausgewählt aus der Gruppe bestehend aus Abu, Ada, Ala, βAla, Arg, Asn, Asp, Bia, Cal, Dab, Gln, Glu, Gly, His, N(im)-A-His, Hph, Ile, Isoser, Leu, tert.-Leu, Lys, Mal, Met, αNal, βNal, Nbg, Nle, Nva, Orn, Phe, Pia, Pro, Pya, Ser, Thr, Tia, Tic, Trp, Tyr und Val, |
| Y | -CN, -$NO_2$, -$CH_2$-$NR^{12}R^{13}$, -$CH_2$-$NR^{12}$-$SO_2R^{14}$, -$CH_2$-$NR^{12}$-$COR^{14}$, -$CH_2$-$NR^{12}$-CO-NH-$R^{14}$, -$CH_2$-$NR^{12}$-CS-NH-$R^{14}$ oder -$COR^{16}$, |
| $R^2$, $R^8$, $R^9$ und $R^{13}$ | jeweils H oder A, |
| $R^4$ | (H, $R^{17}$) oder =O, |
| $R^5$ | OH, $OR^7$, $OCOR^7$, $OSiR^{18}R^{19}R^{20}$, $NR^8R^9$ oder -O-(2-tetrahydropyranyl), |
| $R^6$ | H, A, Ar oder Aralkyl, |
| $R^5$ und $R^6$ | zusammen auch (=O), |
| $R^{16}$ | H, OH, $OR^7$, $NR^8R^9$, $OSiR^{18}R^{19}R^{20}$ oder -O-(2-tetrahydropyranyl), |
| $R^{17}$ | OH oder $NH_2$, |
| $R^5$ und $R^{17}$ | zusammen auch -$T^1$-($CR^3R^7$)-$T^2$-, |

| | |
|---|---|
| $R^3$, $R^7$, $R^{11}$, $R^{12}$ und $R^{14}$ | jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkyl-alkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen, |
| $R^{11}$ | auch $R^8O$-, $R^8R^9N$-, $R^8OOC$- oder $A_3N^{\oplus}$ $An^{\ominus}$, |
| $R^{18}$, $R^{19}$ und $R^{20}$ | jeweils A, Ar oder Aralkyl, |
| $R^{10}$ | H, A oder CN, |
| L | CH oder N, |
| T, $T^1$ und $T^2$ | jeweils O oder $NR^6$, |
| V | $CHOR^2$, CO, S, SO oder $SO_2$, |
| $R^8R^9N$ und $NR^{12}R^{13}$ | auch jeweils eine unsubstituierte oder eine durch A, OH, $NH_2$, NHA, $NA_2$, NHAc, NH-CO-$C_xH_{2x}$-O-$R^{15}$, NH-CO-O-$C_xH_{2x}$-$R^{15}$, Hydroxyalkyl, COOH, COOA, $CONH_2$, Aminoalkyl, HAN-alkyl, $A_2N$-alkyl, $A_3N^{\oplus}$alkyl $An^{\ominus}$, NH-CO-$NH_2$, NH-CO-NHA, Guanidinyl oder Guanidinyl-alkyl substituierte Pyrrolidino-, Piperidino-, Morpholino- oder Piperazinogruppe, |
| $R^{15}$ | A oder Ar-alkyl, |
| s und y | jeweils 0 oder 1, |
| m, n, p, r und x | jeweils 0, 1, 2, 3, 4, 5, 6, 7, 8, 9 oder 10, |
| Ar | unsubstituiertes oder ein- oder mehrfach durch A, OA, Hal, $CF_3$, OH, $NO_2$, Hydroxyalkyl, $NH_2$, NHA, $NA_2$, NHAc, SA, SO-A, $SO_2$-A, $SO_2NH_2$, $SO_2NHA$, COOH, COOA, $CONH_2$, CN, Aminoalkyl, HAN-alkyl, $A_2N$-alkyl, $A_3N^{\oplus}$alkyl $An^{\ominus}$ und/oder Guanidinyl-alkyl substituiertes Phenyl oder unsubstituiertes Naphthyl, |
| Het | einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, OA, Hal, $CF_3$, OH, $NO_2$, Carbonylsauerstoff, $NH_2$, NHA, $NA_2$, NHAc, SA, SO-A, $SO_2$-A, $SO_2NH_2$, $SO_2NHA$, COOH, COOA, $CONH_2$, CN, NH-$SO_2$-A, Ar, Ar-alkyl, Ar-alkenyl, Hydroxyalkyl, Aminoalkyl, HAN-alkyl und/oder $A_2N$-alkyl substituiert sein kann und/oder dessen N- und/oder S-Heteroatome auch oxydiert sein könnnen, |
| Hal | F, Cl, Br oder J, |
| Ac | A-CO-, Ar-CO-, Ar-alkyl-CO- oder A-NH-CO-, |
| $An^{\ominus}$ | ein Anion, das auch fehlen kann, wenn stattdessen eine in der Verbindung der Formel I enthaltene Carboxygruppe in Form eines Carboxylatanions vorliegt, |
| -alkyl- | eine Alkylengruppe mit 1-8 C-Atomen und |
| A | Alkyl mit 1-8 C-Atomen bedeuten, |

worin ferner an Stelle einer oder mehrerer -NH-CO-Gruppen auch eine oder mehrere -NA-CO-Gruppen stehen können,

mit den Maßgaben, daß

a) im Falle $R^1$ = $R^{11}$-$C_mH_{2m}$-$(T)_s$-V-$C_nH_{2n}$-L($R^7$-$C_pH_{2p}$)-$C_rH_{2r}$-CO- mit V = S, SO oder $SO_2$, und Y = -$CH_2NR^{12}R^{13}$ mit $R^{12}$ = $R^{13}$ = H, $R^5$ dann $OCOR^7$, $OSiR^{18}R^{19}R^{20}$, $NR^8R^9$ oder -O-(2-tetrahydropyranyl) ist,

b) im Falle $R^1$ = $R^{11}$-$C_mH_{2m}$-$(T)_s$-V-$C_nH_{2n}$-L($R^7$-$CH_pH_{2p}$)-$C_rH_{2r}$-CO-mit V = S, SO oder $SO_2$, und Y =$COR^{16}$, $R^{16}$ dann H, OH, $OR^7$, $OSiR^{18}R^{19}R^{20}$ oder -O-(2-tetrahydropyranyl) ist,

sowie deren Salze.

**2.** Verfahren zur Herstellung eines Peptid-Analogen der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt, oder daß man eine Carbonsäure der Formel II

$R^1$-$G^1$-OH    II

worin

$G^1$
(a) fehlt,
(b) Z,
(c) $Z^1$ bedeutet,

oder eines ihrer reaktionsfähigen Derivate mit einer Aminoverbindung der Formel III

$H\text{-}G^2\text{-}NR^2\text{-}CHR^3\text{-}CR^4\text{-}CH_2\text{-}CR^5R^6\text{-}Y$ III

worin
$G^2$
(a) Z bedeutet,
(b) fehlt,
(c) $Z^2$ bedeutet und

$Z^1 + Z^2$ zusammen Z bedeuten,
umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Aminogruppe durch Behandeln mit einem acylierenden Mittel acyliert und/oder zur Herstellung einer Verbindung der Formel I, $R^4$ = (H, OH) oder (H, $NH_2$), ein Aminoketosäurederivat der Formel I, $R^4$ = O, reduziert oder reduktiv aminiert und/oder einen Rest $R^1$ in einen anderen Rest $R^1$ umwandelt und/oder eine Verbindung der Formel I durch Behandeln mit einer Säure in eines ihrer Salze überführt.

**3.** Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

**4.** Pharmazeutische Zubereitung, gekennzeichnet durch einen Gehalt an mindestens einer Verbindung der Formel I und/oder einem ihrer physiologisch unbedenklichen Salze.

**5.** Verwendung von Verbindungen der Formel I oder von deren physiologisch unbedenklichen Salzen zur Herstellung eines Arzneimittels.

**6.** Verwendung von Verbindungen der Formel I oder von deren physiologisch unbedenklichen Salzen bei der Bekämpfung der reninabhängigen Hypertension oder des Hyperaldosteronismus.